(19) Europäisches Patentamt
European Patent Office
Office européen des brevets

(11) EP 0 990 643 B1

(12) EUROPÄISCHE PATENTSCHRIFT

(45) Veröffentlichungstag und Bekanntmachung des Hinweises auf die Patenterteilung:
**25.07.2001 Patentblatt 2001/30**

(51) Int Cl.[7]: **C07C 237/12**, C07C 229/16, C07C 217/84, G01N 21/77, G01N 21/64

(21) Anmeldenummer: **99890307.4**

(22) Anmeldetag: **27.09.1999**

(54) **Lumineszenzindikator zur Bestimmung von Calciumionen**

Luminescence indicator for determining calcium ions

Indicateur de luminescence pour la détermination des ions de calcium

(84) Benannte Vertragsstaaten:
**AT DE FR GB**

(30) Priorität: **30.09.1998 US 164516**

(43) Veröffentlichungstag der Anmeldung:
**05.04.2000 Patentblatt 2000/14**

(73) Patentinhaber: **AVL Medical Instruments**
**8207 Schaffhausen (CH)**

(72) Erfinder:
- **He, Huarui**
**Alpharetta, Georgia 30202 (US)**
- **Mortellaro, Mark Alan**
**Woodstock, GA 30188 (US)**

(74) Vertreter: **Schwarz, Albin, Dr. et al**
**Patentanwalt**
**Wipplingerstrasse 32/22**
**1010 Wien (AT)**

(56) Entgegenhaltungen:
**WO-A-96/16327** **US-A- 5 516 911**
- **PATENT ABSTRACTS OF JAPAN vol. 1997, no. 09, 30. September 1997 (1997-09-30) & JP 09 124566 A (FUJI PHOTO FILM CO LTD), 13. Mai 1997 (1997-05-13)**

EP 0 990 643 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft Lumineszenzindikatoren zur Bestimmung von ionisiertem Calcium in einer Probe sowie optische Sensoren, welche diese Lumineszenzindikatoren aufweisen. Die Erfindung betrifft auch ein Verfahren zur Bestimmung von Calciumionen in einer Probe unter Verwendung der erfindungsgemäßen Lumineszenzindikatoren.

Zur Bestimmung der Calciumionen wird die Probe mit einem Lumineszenzindikator (= Luminophor-Ionophor), der einen luminophoren Rest und einen ionophoren Rest aufweist, in zumindest indirekten Kontakt gebracht, welcher ionophore Rest mit den in der Probe enthaltenen Calciumionen reagiert, wonach die Lumineszenz des luminophoren Restes gemessen wird und mit dem Meßergebnis auf die Konzentration oder die Aktivität des ionisierten Calciums geschlossen wird, d.h. das Calciumion bestimmt wird.

Ein derartiges Bestimmungsverfahren beruht auf der reversiblen Bindung von Calciumionen an einen $Ca^{2+}$-selektiven Ionophor und auf dem sogenannten "PET-Effekt" zwischen dem Ionophor und einem luminophoren Rest.

Die reversible Bindung von Calciumionen an den Ionophor erfolgt nach dem Massenwirkungsgesetz (Gleichung 1):

$$(1) \qquad ICa^{2+} \xleftrightarrow{Kd} I + Ca^{2+}$$

wobei, bei gegebener Ionenstärke und Temperatur, die Dissoziationskonstante ($K_d$) durch Gleichung 2 gegeben ist,

$$(2) \qquad \frac{cI\ cCa^{2+}}{cICa^{2+}}$$

wobei I den Ionophor mit der Ladungszahl -2, ICa den Ionophor-Ion-Komplex und *c* die Konzentration darstellen. $K_d$ und $cCa^{2+}$ werden in der Folge in mol/l bzw. mmol/l angegeben.

Der $pK_d$-Wert (Gleichung 3) ist der negative dekadische Logarithmus der Dissoziationskonstante:

$$(3) \qquad pK_d = -\log(K_d)$$

Unter "PET-Effekt" wird ein mittels Photonen induzierter Elektronen-Transfer (photoinduced electron transfer = PET) vom ionophoren Rest bzw. Ionophor auf den luminophoren Rest bzw. Luminophor verstanden, der eine Verringerung der (relativen) Lumineszenzintensität und der Lumineszenzabklingzeit des Luminophors bewirkt. Die Absorptions- und die Emissionswellenlängen des luminophoren Restes bzw. des Luminophors werden dabei im wesentlichen nicht beeinflußt (J.R. Lakowicz in "Topics in Fluorescence Spectroscopy", Band 4: Probe Design and Chemical Sensing; Plenum Press, New York and London (1994)).

Durch die Bindung von Ionen an den Ionophor wird der PET-Effekt reduziert oder vollständig unterdrückt, sodaß es zu einem Anstieg der Lumineszenz des luminophoren Restes kommt. Somit kann durch Messung der Änderung der Lumineszenzeigenschaften, d.h. der Lumineszenzintensität und/oder der Lumineszenzabklingzeit, auf die Konzentration oder die Aktivität des zu bestimmenden Ions geschlossen werden.

Bei Säugetieren übt Calcium wichtige physiologische Funktionen aus. Diese Funktionen schließen ein: (1) Kontrolle der Blutgerinnung durch Aktivierung der Bildung von Thrombin aus Prothrombin, (2) Erregung von Muskel-, Herz- und Nervenzellen, einschließlich der Funktion als sekundärer Botenstoff wie cAMP.

Die Messung von extrazellulärem, ionisiertem Calcium ist ein essentieller Teil der medizinischen Diagnostik. Die Messung von ionisiertem Calcium, Blutgasen und Kalium ist obligatorisch, um die Erhaltung einer guten Herzfunktion während Lebertransplantationen oder anderen Operationen, welche einen Bypass der Herz- und Lungenfunktionen und den Einsatz künstlicher lebenserhaltender Maßnahmen erfordern, zu ermöglichen.

Ionenselektive Elektroden (ISE) werden seit vielen Jahren zur Bestimmung von Calciumionen in Körperflüssigkeiten eingesetzt. Entscheidende Nachteile elektrochemischer Meßanordnungen sind die Notwendigkeit eines Referenzelementes, die Empfindlichkeit gegen elektrische Potentiale und elektromagnetische Störungen. Zwar sind ionenselektive Elektroden robust und verläßlich, jedoch ist ihre Verwendung in einer Vorrichtung zur einmaligen Anwendung teuer. Außerdem benötigen diese Elektroden eine elektrische Verbindung der Probenmeßvorrichtung zum Instrument.

Optische Methoden bzw. optische Sensoren hingegen benötigen kein Referenzelement. Die optischen Signale sind unabhängig von externen Potentialen und Stromflüssen. Bisher bekannt gewordene optische Methoden

zur Bestimmung von Calcium beruhen beispielsweise auf der Messung der direkt oder indirekt von der Konzentration bzw. Aktivität von Calciumionen abhängigen Lumineszenzintensität oder Lumineszenzabklingzeit eines Calcium-spezifischen Lumineszenzindikators bzw. der Lichtabsorption eines Calcium-spezifischen Absorptionsindikators.

Zur Bestimmung intrazellulärer Calcium-Konzentrationen werden beispielsweise Indikatoren eingesetzt, welche ihre Absorptions- und/oder Lumineszenzeigenschaften durch reversible Bindung von Calciumionen verändern (siehe oben, Gleichung 1). Geeignete Indikatoren für intrazelluläre Calcium-Bestimmungen beruhen beispielsweise auf $Ca^{2+}$-komplexierenden Tetracarboxylat-Verbindungen, welche die achtfach koordinativen Ligandengruppenmerkmale von EGTA (= Ethylenglycol-bis(beta-aminoethylether)-N,N,N',N'-tetraessigsäure) und BAPTA (= 1,2-bis(2-Aminophenoxy)ethan-N,N,N',N'-tetraessigsäure) aufweisen.

So offenbart beispielsweise die US-A - 4 603 209 BAPTA-Analoge, welche elektronisch an ein oder zwei, im UV-Bereich anregbare Fluoreszenzfarbstoffmoleküle gekoppelt werden. Durch die Bindung von Calciumionen an den Ionophor wird die Absorptionswellenlänge des Farbstoffs (ein Stilbenderivat) verändert. Durch geeignete elektronenziehende bzw. elektronengebende Substituenten kann die Calcium-Dissoziationskonstante im Bereich von 80 bis 250 nmol/l verändert werden.

Weiters beschreibt die US-A - 5 049 673 BAPTA-Analoge, welche - elektronisch entkoppelt - an Xanthen-Farbstoffe (Fluoresceine, Rhodamine) gebunden werden. Durch die elektronische Entkopplung vom Fluorophor tritt ein PET-Effekt ein, erkennbar an der konstanten spektralen Lage der Fluoreszenzemissionsbande und der steigenden Fluoreszenzintensität in Abhängigkeit von steigenden $Ca^{2+}$-Konzentrationen (siehe Fig. 4a des Dokuments). Aufgrund der niedrigen $K_d$-Werte ist ersichtlich, daß die beschriebenen Fluoroionophore (siehe Fig. 6 des Dokuments) nicht zur Bestimmung millimolarer $Ca^{2+}$-Konzentrationen im Vollblut bzw. Blutplasma geeignet sind.

Bedingt durch die sehr niedrige Dissoziationskonstante sind derartige Calcium-Ionophore zur Bestimmung von $Ca^{2+}$ in Proben mit entsprechend niedrigen Calciumwerten, wie z.B. intrazellulärem $Ca^{2+}$, geeignet. Hingegen weist beispielsweise Blutplasma $Ca^{2+}$-Konzentrationen im Bereich von ca. 0,4-2 mmol/l auf. Geeignete Ionophore zur optischen Bestimmung von $Ca^{2+}$ im Blut bzw. Blutplasma müssen daher entsprechend hohe $K_d$-Werte aufweisen. Ideale $K_d$-Werte liegen im Erwartungsbereich der zu bestimmenden $Ca^{2+}$-Konzentrationen bzw. -Aktivitäten.

Aus der US-A - 5 516 911 sind Fluoreszenzindikatoren basierend auf fluorierten BAPTA-Derivaten bekannt, welche $K_d$-Werte im millimolaren Bereich aufweisen. Nachteilig ist hier vor allem die sehr aufwendige Synthese fluorierter BAPTA-Derivate.

Die bekannten Ionophore basierend auf BAPTA bzw. dessen Derivaten sind außerdem in wäßriger Umgebung und bei normalen Umgebungstemperaturen chemisch nicht besonders stabil (siehe US-A - 4 603 209, Spalte 26, Zeilen 40-46). Dies ist insbesondere bei Bestimmungen mit optischen Sensoren bei jenen Meßsituationen nachteilig, wo eine hohe Lebensdauer (Haltbarkeit) des Sensors erforderlich ist bzw. zum Zwecke eines Monitorings über längere Zeiträume mit einem Sensor gemessen werden soll.

Die vorliegende Erfindung bezweckt die Vermeidung dieser Nachteile und Probleme und stellt sich die Aufgabe, Luminophor-Ionophore zur optischen Bestimmung von Calciumionen bereitzustellen, deren Ionophore im Vergleich zu bisher bekannten BAPTA-Verbindungen, insbesondere fluorierten Derivaten, einfacher zu synthetisieren sind und - elektronisch entkoppelt - kovalent an geeignete Luminophore gebunden werden können.

Die Ionophore der bereitgestellten Luminophor-Ionophore sollen ferner $K_d$-Werte aufweisen, welche ohne vorherige Verdünnungsschritte des Probenmaterials die Bestimmung physiologischer Calciumwerte erlauben, wobei die $K_d$-Werte durch geeignete Substituenten hinsichtlich der Erwartungswerte der zu bestimmenden $Ca^{2+}$-Konzentrationen des Probenmaterials einstellbar sein sollen.

Die Luminophor-Ionophore sollen außerdem für ihre Verwendung in optischen Sensoren mittels einer chemischen Gruppe an ein hydrophiles Polymermaterial gebunden werden können.

Bevorzugte Luminophor-Ionophore sollen im Erwartungsbereich der pH-Werte der Probe keine inhärente pH-Abhängigkeit aufweisen und mit Licht kommerziell erhältlicher LED's (vorzugsweise >420 nm) anregbar sein. Diese Luminophor-Ionophore sollen außerdem in wäßriger Umgebung auch bei hohen Umgebungstemperaturen und über längere Zeiträume (>3 Monate) chemisch stabil sein.

Die vorliegende Aufgabe wird dadurch gelöst, daß eine Verbindung mit der allgemeinen Formel I

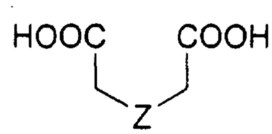

(I)

einschließlich deren Salze bereitgestellt wird, worin Z entweder die Gruppe mit der allgemeinen Formel II

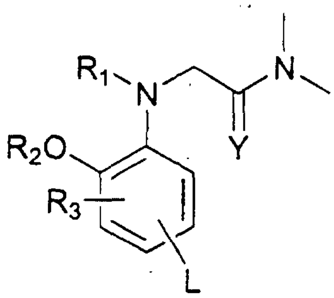

(II)

ist, in welcher

- $R_1$ Alkyl mit 1-4 C-Atomen, Alkoxyalkyl mit 2-5 C-Atomen oder Aryloxyalkyl, dessen Alkylrest 1-4 C-Atome aufweist, ist,
- $R_2$ Alkyl mit 1-4 C-Atomen oder Alkoxyalkyl mit 2-5 C-Atomen ist,
- $R_3$ H, Alkoxy mit 1-4 C-Atomen, Halogen, NO oder $NO_2$ ist,
- Y $H_2$ oder O ist und
- L ein luminophorer Rest in para- oder meta-Stellung zum Stickstoff ist,

oder die Gruppe mit der allgemeinen Formel III

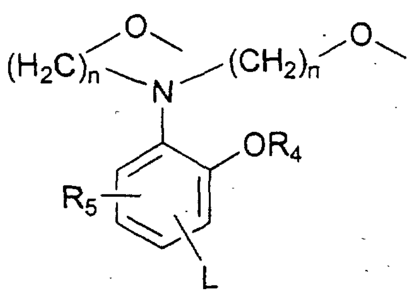

(III)

ist, in welcher

- n 2 oder 3 ist,
- $R_4$ Alkyl mit 1-4 C-Atomen oder Alkoxyalkyl mit 2-5 C-Atomen ist,
- $R_5$ H, Alkoxy mit 1-4 C-Atomen, Halogen, NO oder $NO_2$ ist und
- L ein luminophorer Rest in para- oder meta-Stellung zum Stickstoff ist,

oder die Gruppe mit der allgemeinen Formel IV

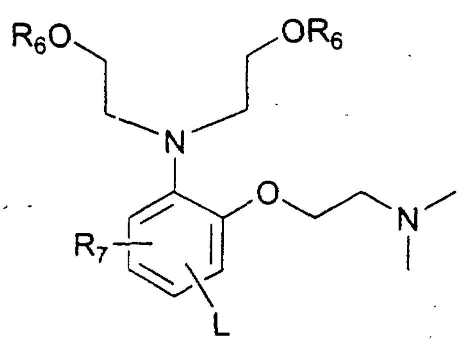

(IV)

ist, in welcher

$R_6$ Alkyl mit 1-3 C-Atomen oder Phenyl ist,
$R_7$ H, Alkoxy mit 1-4 C-Atomen, Halogen, NO oder $NO_2$ ist und
L ein luminophorer Rest in para- oder meta-Stellung zum Stickstoff ist.

Die erfindungsgemäßen Verbindungen weisen daher die folgenden allgemeinen Formeln

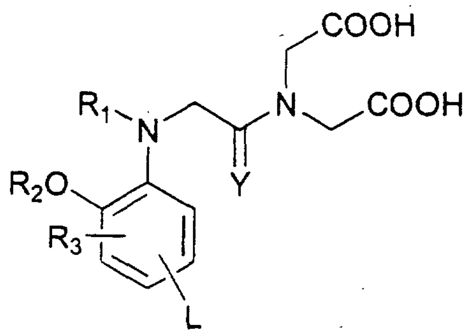

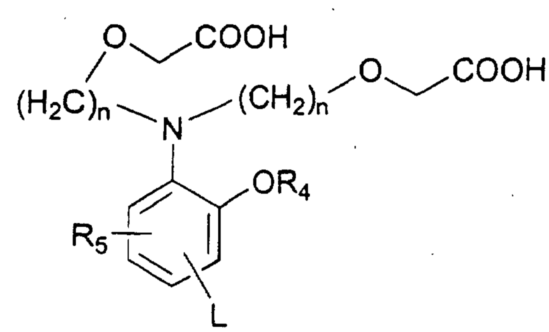

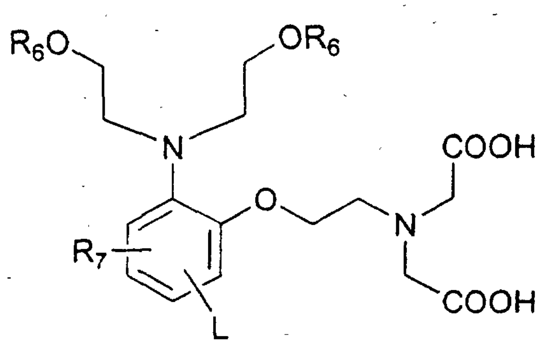

auf, wobei $R_1$, $R_2$, $R_3$, $R_4$, $R_5$, $R_6$, $R_7$, Y, L und n die oben angegebene Bedeutung besitzen. Die Ionophore der erfindungsgemäßen Verbindungen basieren auf der Grundstruktur des o-Anisidins mit einem Diacetatrest und können auf einfache Weise synthetisiert werden (siehe unten).

Die $K_d$-Werte der erfindungsgemäßen Ionophore liegen in einem Bereich, der sie insbesondere zur Bestimmung physiologischer $Ca^{2+}$-Konzentrationen geeignet macht (siehe Beispiele in Tabelle 1: $K_d$ = 0,2-123 mmol/l $Ca^{2+}$). Die $K_d$-Werte können durch geeignete Substituenten eingestellt werden (siehe unten).

Infolge des aliphatisch gebundenen Stickstoffs des Imino-N,N-Diacetat-Ligandenteils weisen Ionophore der Verbindungen mit einer Gruppe der allgemeinen Formel II bei neutralen pH-Werten eine inhärente pH-Abhängigkeit auf und sind in wäßriger Umgebung über längere Zeit nicht stabil. Grundsätzlich können diese Ionophore jedoch für jede Meßsituation eingesetzt werden, wo der pH-Wert der Probe bekannt ist oder mittels eines pH-Puffers auf einen bekannten Wert eingestellt werden kann und eine längere Lebensdauer in wäßriger Umgebung nicht erforderlich ist.

Bei den erfindungsgemäßen Verbindungen mit einer Gruppe der allgemeinen Formel II ist $R_1$ bevorzugt Alkyl mit 4 C-Atomen, Alkoxyalkyl mit 3-4 C-Atomen oder Aryloxyalkyl, dessen Alkylrest 2 C-Atome aufweist. $R_2$ ist bevorzugt Alkyl mit 1-4 C-Atomen, insbesonders bevorzugt Methyl. $R_3$ ist vorteilhafterweise in para-Stellung zum o-Anisidin-Sauerstoff angeordnet und ist bevorzugt H oder Methoxy.

Die besonders vorteilhaften Ionophore der Verbindungen mit einer Gruppe der allgemeinen Formel III weisen bei physiologischen pH-Werten (vermutlich auch alkalisch) keine inhärente pH-Abhängigkeit auf und sind in wäßriger Umgebung bei normaler Umgebungstemperatur auch über längere Zeiträume chemisch stabil. Diese Ionophore basieren ebenfalls auf der Struktur des o-Anisidins und einem Diacetat. Im Gegensatz zu den Ionophoren mit einer Gruppe der allgemeinen Formel II weisen sie jedoch keinen aliphatisch gebundenen Stickstoff auf. Die beiden Carboxyl-Ligandenteile sind in Form von Diethoxyacetat-Gruppen an den aromatischen Stickstoff des o-Anisidins gebunden.

Bei den erfindungsgemäßen Verbindungen mit einer Gruppe der allgemeinen Formel III ist es bevorzugt, daß

n = 2 ist. $R_4$ ist vorzugsweise Alkyl mit 1-2 C-Atomen. $R_5$ ist vorteilhafterweise in para-Stellung zum o-Anisidin-Sauerstoff angeordnet und ist bevorzugt H oder Ethoxy oder Cl.

Die Ionophore der Verbindungen mit einer Gruppe der allgemeinen Formel IV weisen im Vergleich zu Ionophoren mit einer Gruppe der allgemeinen Formel III ähnliche Vorteile auf.

Bei den erfindungsgemäßen Verbindungen mit einer Gruppe der allgemeinen Formel IV ist $R_6$ vorzugsweise Methyl. $R_7$ ist vorteilhafterweise in para-Stellung zum o-Anisidin-Sauerstoff angeordnet und ist bevorzugt H.

Geeignete luminophore Reste L können chemisch via $-(CH_2)_n$ - Spacer an die erfindungsgemäßen Ionophore gebunden werden, wobei n vorzugsweise die Zahlen 1 und 2 bedeutet, aber auch 0 sein kann. Als luminophore Reste sind grundsätzlich alle luminophoren Reste geeignet, welche in Kombination mit den erfindungsgemäßen Ionophoren einen PET-Effekt (siehe oben) ergeben.

Wie dem Fachmann bekannt ist, ist für das Zustandekommen eines PET-Effektes insbesondere eine elektronische Entkopplung des Elektronendonors des ionophoren Restes vom elektronischen System des luminophoren Restes wesentlich. Diese elektronische Entkopplung von ionophorem und luminophorem Rest kann bekanntermaßen dadurch erreicht werden, daß beide Reste entweder durch eine Spacer-Gruppe, also z.B. eine $(CH_2)_n$-Kette oder - wenn n=0 - durch einen virtuellen Spacer (z.B. durch Verdrehung der Ebene des luminophoren Restes zur Ebene des Benzolrings) getrennt vorliegen. Die Funktion des Spacers besteht somit darin, einer Konjugation des Elektronensystems des ionophoren Restes mit dem Elektronensystem des luminophoren Restes entgegenzuwirken.

Die elektronische Entkopplung ist z.B. daran erkenntlich, daß sich die Absorptions- und Emissionsspektren des luminophoren Restes hinsichtlich ihrer Wellenlänge in Abhängigkeit von der Calciumkonzentration nicht wesentlich ändern.

Bevorzugt sind solche luminophoren Reste, welche mit Licht kommerziell erhältlicher LED's angeregt werden können, keine inhärente pH- (und $O_2$-) Empfindlichkeit aufweisen und in Form der Luminophor-Ionophor-Verbindung über längere Zeit in wäßriger Umgebung stabil sind. Ferner soll es möglich sein, am luminophoren oder am ionophoren Rest, vorzugsweise aber am Luminophor, chemische Gruppen anzubringen, anhand derer der Luminophor-Ionophor vorzugsweise kovalent an eine hydrophile Polymermatrix gebunden werden kann.

Beispiele für geeignete luminophore Reste sind lumineszierende Derivate des Naphthalimids, der Difluorobora-3a,4a-s-indacene und der Xanthenone (beispielsweise Fluoresceine und Rhodamine).

Bei den erfindungsgemäßen Verbindungen ist der luminophore Rest L bevorzugt in para-Stellung zum Stickstoff angeordnet.

Die erfindungsgemäßen Verbindungen mit der allgemeinen Formel I können als freie Dicarbonsäuren oder in Form von deren Salzen vorliegen. Vorteilhafterweise liegen die Verbindungen in Form des Dikaliumsalzes vor.

Die Erfindung betrifft auch einen optischen Sensor zur Bestimmung von Calciumionen in einer Probe, welcher Sensor eine Matrix, die eine Verbindung mit einem luminophoren Rest und einem ionophoren Rest aufweist, besitzt, wobei als Verbindung eine Verbindung mit einer Gruppe der allgemeinen Formel I eingesetzt wird.

Die Erfindung stellt ferner ein Verfahren zur Bestimmung von Calciumionen in einer Probe zur Verfügung, wobei die Calciumionen mit einer Verbindung, die einen luminophoren Rest und einen ionophoren Rest aufweist, in zumindest indirekten Kontakt gebracht werden, welcher ionophore Rest mit den in der Probe enthaltenen Caiciumionen reagiert, wobei der luminophore Rest seine Lumineszenzeigenschaften ändert, wonach die Lumineszenz gemessen und mit dem Meßergebnis die Calciumionen bestimmt werden, wobei als Verbindung eine Verbindung mit einer Gruppe der allgemeinen Formel I eingesetzt wird.

Die Erfindung betrifft außerdem die Verwendung einer Verbindung mit einer Gruppe der allgemeinen Formel I in einem optischen Sensor zur Bestimmung von Calciumionen in einer Probe.

Nachfolgend wird die Erfindung beispielhaft noch näher beschrieben, wobei Synthese und Eigenschaften einiger bevorzugt verwendeter Verbindungen erläutert werden. Andere erfindungsgemäße Verbindungen können vom Fachmann auf analoge Weise hergestellt werden.

1. Synthese von erfindungsgemäßen Ionophoren

Figur 1 zeigt die Formeln der in den folgenden Beispielen synthetisierten erfindungsgemäßen Calcium-Ionophore **(X1 - X9).**

Die Synthese der erfindungsgemäßen Calcium-Ionophore kann in drei allgemeine Synthesetrategien eingeteilt werden.

Die erste Synthesestrategie basiert auf einer Monoalkylierung des o-Anisidins, welches dann mit Diethyl-N-(2-bromethyl)iminodiacetat **(M9)** alkyliert wird. Die Verbindungen **X2, X3, X4** und **X5** gehören zu dieser Kategorie. Das Syntheseschema für diese Verbindungen ist in Figur 2B dargestellt.

Die zweite Synthesestrategie basiert auf einer Dialkylierung von o-Alkoxyanilinen. Das Alkylierungsmittel für die Verbindungen **X6, X7** und **X8** war Ethyl-2-chlorethoxyacetat (**M25**). Das Syntheseschema für diese Verbindungen ist in Figur 2C dargestellt.

Die dritte Synthesestrategie, welche die Verbindungen **X1** und **X9** einschließt, verwendete nicht das gleiche Zwischenprodukt. Diese Verbindungen wurden nach individuellen Syntheseschemata hergestellt, welche in Figur 2A dargestellt sind.

1.1. Synthese von Kalium-N-[2-(N',N'-dimethoxyethyl)aminophenoxyethyl]imino-N,N-diacetat **(X9,** siehe Figur 2A)

1.1.1 2-(2-Bromethoxy)-nitrobenzol **(M2)**

Eine Suspension von 35,4 g (200 mmol) Kalium-2-nitrophenolat, 112,7 g (600 mmol) 1,2-Dibromethan in 100 ml DMF wurde 3 Stunden auf 120°C erhitzt. Das Gemisch wurde gekühlt und mit 400 ml $CHCl_3$ verdünnt, mit 3x400 ml 2,5%igem $Na_2CO_3$ gewaschen, bis die wäßrige Phase nahezu farblos war, dann mit 400 ml gesättigtem NaCl gewaschen und über $Na_2SO_4$ getrocknet. Das Lösungsmittel wurde abgedampft und der Rückstand wurde mit 50 ml Methanol zerstoßen, der erhaltene Niederschlag wurde abfiltriert (Dimer). Das Filtrat wurde eingeengt, wobei 22,7 g Öl erhalten wurden, welches nach Abkühlen auf Raumtemperatur erstarrte. Nach Umkristallisieren aus Methanol/Wasser (95/5, v/v) wurden 17,8 g weißliche Kristalle erhalten (Ausbeute: 36,2%).
$^1$H-NMR ($CDCl_3$), δ (ppm): 3,68 (t, 2H), 4,42 (t, 2H), 7,08 (m, 2H), 7,55 (m, 1H), 7,83 (m, 1H).

1.1.2. Diethyl-N-(2-nitrophenoxyethyl)imino-N,N-diacetat **(M3)**

Ein Gemisch von 2,46 g (10 mmol) **M2,** 2,08 g (11 mmol) Diethyliminodiacetat **M8** (siehe Figur 2B), 1,52 g (11 mmol) $K_2CO_3$, 0,83 g (5 mmol) KI und 10 ml DMF wurde 20 Stunden auf 85°C erhitzt, gekühlt und mit 50 ml $CHCl_3$ und 50 ml Wasser verdünnt. Die organische Phase wurde mit 50 ml gesättigtem NaCl gewaschen und über $Na_2SO_4$ getrocknet. Das Lösungsmittel wurde abgedampft, wobei 4,10 g Rohöl erhalten wurden. Dieses Öl wurde mittels einer Silicagelsäule mit $CHCl_3$ und Cyclohexan als Eluierungsmittel gereinigt, wobei 2,68 g klares Öl erhalten wurden (Ausbeute: 76%).
$^1$H-NMR ($CDCl_3$), δ (ppm): 1,25 (t, 6H), 3,25 (t, 2H), 3,69 (s, 4H), 4,14 (m, 4H), 4,27 (t, 2H), 6,98-7,10 (m, 2H), 7,51 (m, 1H), 7,84 (m, 1H).

1.1.3. Diethyl-N-(2-aminophenoxyethyl)imino-N,N-diacetat **(M4)**

2,60 g (7,3 mmol) **M3** und 0,26 g 10% Pd/C wurden in 50 ml absolutem Ethanol suspendiert, 2 Stunden bei 30 psi hydrogeniert und filtriert. Das Lösungsmittel wurde abgedampft, wobei 2,20 g hellgelbes Öl erhalten wurden (Ausbeute: 92%).
$^1$H-NMR ($CDCl_3$), δ (ppm): 1,25 (t, 6H), 3,20 (t, 2H), 3,65 (s, 6H, inkl. $NH_2$), 4,18 (m, 6H), 4,27 (t, 2H), 6,70-6,80 (m, 4H, Ar.).

1.1.4. Diethyl-N-[2-(N',N'-dimethoxyethyl)aminophenoxyethyl]imino-N,N-diacetat **(M5)**

Eine Suspension von 1,62 g (5 mmol) **M4,** 23,5 g (350 mmol) Chlorethylmethylether, 1,65 g (12 mmol) $K_2CO_3$, 1,0 g (6 mmol) KI in 25 ml DMF wurde 18 Stunden auf 95 °C erhitzt. Der größte Teil des DMF und des nicht umgesetzten Chlorethylmethylethers wurden abgedampft. Der Rückstand wurde in 100 ml $CHCl_3$ und 100 ml gesättigtem NaCl gelöst. Die organische Phase wurde über $Na_2SO_4$ getrocknet. Das Lösungsmittel wurde abgedampft, wobei 2,21 g Öl erhalten wurden. Dieses Rohöl wurde mit Silicagel 100 unter Verwendung von $CHCl_3$ als Eluierungsmittel gereinigt, wobei 0,64 g Reinprodukt erhalten wurden (Ausbeute: 29%).
$^1$H-NMR ($CDCl_3$), δ (ppm): 1,23 (t, 6H), 3,20 (t, 2H), 3,32 (t, 2H), 3,40 (t, 4H), 3,60 (t, 2H), 3,65 (s, 4H), 4,18 (m, 6H), 6,60-6,85 (m, 4H, Ar.).

1.1.5. Kalium-N-[2-(N',N'-dimethoxyethyl)aminophenoxyethyl]imino-N,N-diacetat **(X9)**

0,25 ml Wasser und 0,09 g (1,5 mmol) KOH wurden einer Lösung von 0,22 g (0,5 mmol) **M5** in 4,75 ml Methanol hinzugefügt. Die erhaltene Lösung wurde 5 min auf 60°C erhitzt und dann 4 Stunden bei Raumtemperatur gerührt. Dünnschichtchromatographie zeigte, daß der Ester vollständig hydrolysiert war. Die Konzentration an **X9** in dieser endgültigen Lösung betrug 100 mmol/l. 5 ml dieser Lösung wurden mit HEPES-Puffer (pH 7,3), welcher 140 mmol/l $Na^+$, 5 mmol/l $K^+$ und 110 mmol/l $Cl^-$ enthielt, auf 1000 ml verdünnt. Die in Figur 4 dargestellten UV-Absorptionskurven wurden mit dieser Lösung unter Zusatz verschiedener Konzentrationen von $CaCl_2$ gemessen.

1.2. Synthese von Kalium-N-[2-(N'-methoxyethyl-N'-(2-methoxyphenyl)aminoacetyl)]-imino-N,N-diacetat (**X1**, siehe Figur 2A)

1.2.1. Diethyl-N-(bromacetyl)imino-N,N-diacetat **(M6)**

Eine Lösung von 9,44 g (60 mmol) Bromacetylchlorid in 25 ml $CH_2Cl_2$ wurde einer Lösung von 9,5 g (50 mmol) Diethyliminodiacetat **M8** (siehe Figur 2B) und 6,07 g (60 mmol) Triethylamin in 25 ml $CH_2Cl_2$ bei 0°C hinzugefügt. Die erhaltene Suspension wurde 4 Stunden bei Raumtemperatur gerührt, mit 150 ml $CHCl_3$ verdünnt und mit 200 ml Wasser, 3 x 200 ml 0,1 N HCl, 3x200 ml gesättigtem $NaHCO_3$ und 200 ml gesättigtem NaCl gewaschen und über $Na_2SO_3$ getrocknet. Das Lösungsmittel wurde abgedampft, wobei 12,6 g Öl erhalten wurden. Dieses Öl wurde mittels einer Silicagelsäule unter Verwendung von $CHCl_3$/Cyclohexan (1/1, v/v) als Eluierungsmittel gereinigt, wobei 9,25 g Reinprodukt erhalten wurden (Ausbeute: 60%). $^1$H-NMR ($CDCl_3$), δ (ppm): 1,25 (t, 6H), 2,10 (s, 2H), 4,20 (m, 8H).

1.2.2. Diethyl-N-[2-(N'-methoxyethyl-N'-(2-methoxyphenyl)aminoacetyl)]-imino-N,N-diacetat **(M7)**

Eine Suspension von 0,73 g (4 mmol) **M12** (Synthese siehe 1.3.2. und Figur 2B), 1,86 g (6 mmol) **M6** und 0,83 g (6 mmol) $K_2CO_3$ in 6 ml Acetonitril wurde 18 Stunden auf 85°C erhitzt. Das Gemisch wurde gekühlt und mit 30 ml $CHCl_3$ und 30 ml Wasser verdünnt. Die organische Phase wurde mit 30 ml gesättigtem NaCl gewaschen und über $Na_2SO_4$ getrocknet. Das Lösungsmittel wurde abgedampft, wobei 2,55 g Rohprodukt erhalten wurden. Dieses Öl wurde mittels einer mit 13 g Silicagel gepackten Säule gereinigt und mit $CHCl_3$ zur Gewinnung des Reinprodukts eluiert, wobei 0,96 g erhalten wurden (Ausbeute 59%).
$^1$H-NMR ($CDCl_3$), δ (ppm): 1,25 (t, 6H), 3,40 (t, 2H), 3,50 (t, 2H), 3,80 (s, 3H), 4,05 (s, 3H), 4,08 (s, 4H), 4,12 (q, 4H), 4,50 (s, 2H), 6,80-7,10 (m, 4H, Ar.).

1.2.3. Kalium-N-[2-(N'-methoxyethyl-N'-(2-methoxyphenyl)aminoacetyl)]-imino-N,N-diacetat **(X1)**

Ein ähnliches Verfahren wie das oben in Abschnitt 1.1.5. beschriebene wurde verwendet, um **M7** zu hydrolysieren und eine Lösung von **X1** zu erhalten, welche für Absorptionsmessungen verwendet wurde.

1.3. Synthese von Kalium-N-[2-(N'-methoxyethyl-N'-(2-methoxyphenyl)aminoethyl)]-imino-N,N-diacetat (**X2**, siehe Figur 2B)

1.3.1. Diethyl-N-(2-bromethyl)imino-N,N-diacetat **(M9)**

Eine Lösung von 9,5 g (50 mmol) Diethyliminodiacetat **M8**, 94,0 g (500 mmol) 1,2-Dibromethan und 7,8 g (60 mmol) Diisopropylethylamin wurde 18 Stunden auf 85°C erhitzt. Dann wurde sie gekühlt und mit 100 ml $CHCl_3$ und 100 ml Wasser verdünnt. Die organische Phase wurde mit 2x200 ml Wasser und 200 ml gesättigtem NaCl gewaschen und über $Na_2SO_4$ getrocknet. Lösungsmittel und nicht umgesetztes Dibromethan wurden abgedampft, der Rückstand wurde mittels einer Silicagelsäule unter Verwendung von $CHCl_3$/Cyclohexan (1/1, v/v) als Eluierungsmittel gereinigt. Es wurden 4,0 g klares Öl erhalten (Ausbeute: 27%).
$^1$H-NMR ($CDCl_3$), δ (ppm): 1,23 (t, 6H), 3,15 (t, 2H), 3,40 (t, 2H), 3,60 (s, 4H), 4,08 (m, 4H).

1.3.2. N-Methoxyethyl-2-anisidin (**M12**)

Eine Suspension von 24,6 g (200 mmol) o-Anisidin **M10,** 20,8 g (220 mmol) Chlorethylmethylether **M11,** 30,4 g (220 mmol) $K_2CO_3$ und 18,3 g (110 mmol) KI in 110 ml DMF wurde 24 Stunden auf 95 °C erhitzt. DMF wurde abgedampft, und der Rückstand wurde mit 100 ml $CHCl_3$ aufgeschlossen, filtriert und der Niederschlag mit 2x50 ml $CHCl_3$ gewaschen. Das Lösungsmittel wurde abgedampft, wobei 41,2 g Rohöl erhalten wurden. Dieses Öl wurde mittels eines mit 184 g Silicagel 100 gepackten Plugs unter Verwendung von $CHCl_3$/Cyclohexan (1:1, v/v) als Eluierungsmittel gereinigt, wobei 23,5 g Reinprodukt erhalten wurden (Ausbeute: 65%).
$^1$H-NMR ($CDCl_3$), δ (ppm): 3,28 (t, 2H), 3,40 (s, 3H), 3,65 (t, 2H), 3,78 (s, 3H), 6,35 (d, 1H), 6,60 (t, 1H), 6,78 (m, 2H).

1.3.3. Diethyl-N-[2-(N'-methoxyethyl-N'-(2-methoxyphenyl)aminoethyl)]-imino-N,N-diacetat **(M13)**

Eine Suspension von 0,36 g (2 mmol) **M12**, 0,65 g (2,2 mmol) **M9** und 0,30 g (2,2 mmol) $K_2CO_3$ in 2 ml DMF wurde 18 Stunden auf 85 °C erhitzt. Das Gemisch wurde gekühlt und mit 20 ml $CHCl_3$ und 20 ml Wasser verdünnt. Die organische Phase wurde mit 20 ml gesättigtem NaCl gewaschen und über $Na_2SO_4$ getrocknet. Das Lösungsmittel wurde abgedampft, wobei 1,05 g Rohprodukt erhalten wurden. Dieses Öl wurde mittels einer mit 5 g Silicagel gepackten

Säule gereinigt, wobei zur Entfernung von nicht-umgesetztem **M12** mit $CHCl_3$ und zur Gewinnung des Reinprodukts mit $CHCl_3$/Ethylacetat (4/1, v/v) eluiert wurde. Es wurden 0,33 g Reinprodukt erhalten (Ausbeute: 51%).
$^1$H-NMR ($CDCl_3$), δ (ppm): 1,25 (t, 6H), 2,85 (t, 2H), 3,30 (s, 3H), 3,35 (m, 4H), 3,40 (t, 2H), 3,60 (s, 4H), 3,80 (s, 3H), 4,18 (q, 4H), 6,80-7,05 (m, 4H, Ar.).

1.3.4. Kalium-N-[2-(N'-methoxyethyl-N'-(2-methoxyphenyl)aminoethyl)]-imino-N,N-diacetat (**X2**)

Ein ähnliches Verfahren wie das oben in Abschnitt 1.1.5. beschriebene wurde verwendet, um **M13** zu hydrolysieren und eine Lösung von **X2** zu erhalten, welche für Absorptionsmessungen verwendet wurde.

1.4. Synthese von Kalium-N-[2-(N'-ethoxyethyl-N'-(2-methoxyphenyl)aminoethyl)]-imino-N,N-diacetat **(X3,** siehe Figur 2B)

1.4.1. N-Ethoxyethyl-2-anisidin **(M15)**

Eine Suspension von 12,3 g (100 mmol) o-Anisidin **M10**, 11,9 g (110 mmol) Chlorethylethylether **M14,** 15,2 g (110 mmol) $K_2CO_3$ und 9,1 g (55 mmol) KI in 55 ml DMF wurde 18 Stunden auf 95 °C erhitzt. DMF wurde abgedampft, und der Rückstand wurde in 100 ml $CHCl_3$ und 100 ml gesättigtem NaCl gelöst. Die organische Phase wurde über $Na_2SO_4$ getrocknet. Das Lösungsmittel wurde abgedampft, wobei 19,4 g Rohöl erhalten wurden. Das Rohöl wurde mittels eines mit 84 g Silicagel 100 gepackten Plugs unter Verwendung von $CHCl_3$/Cyclohexan (1:1, v/v) als Eluierungsmittel gereinigt, wobei 7,85 g Reinprodukt erhalten wurden (Ausbeute: 40%).
$^1$H-NMR ($CDCl_3$), δ (ppm): 1,25 (t, 3H), 3,30 (t, 2H), 3,58 (q, 2H), 3,65 (t, 2H), 3,78 (s, 3H), 6,35 (d, 1H), 6,60 (t, 1H), 6,78 (m, 2H).

1.4.2. Diethyl-N-[2-(N'-ethoxyethyl-N'-(2-methoxyphenyl)aminoethyl)]-imino-N,N-diacetat **(M16)**

Eine Suspension von 0,59 g (3 mmol) **M15**, 1,33 g (4,5 mmol) **M9** (Synthese siehe 1.3.1.) und 0,62 g (4,5 mmol) $K_2CO_3$ in 3 ml DMF wurde 18 Stunden auf 85 °C erhitzt. Das Gemisch wurde gekühlt und mit 50 ml $CHCl_3$ und 20 ml Wasser verdünnt. Die organische Phase wurde mit 50 ml gesättigtem NaCl gewaschen und über $Na_2SO_4$ getrocknet. Das Lösungsmittel wurde abgedampft, wobei 1,25 g Rohprodukt erhalten wurden. Dieses Öl wurde mittels einer mit 6 g Silicagel gepackten Säule gereinigt, wobei zur Entfernung von nicht-umgesetztem **M15** mit $CHCl_3$ und zur Gewinnung des Reinprodukts mit $CHCl_3$/Ethylacetat (4/1, v/v) eluiert wurde. Es wurden 0,25 g Reinprodukt erhalten (Ausbeute: 26%).
$^1$H-NMR ($CDCl_3$), δ (ppm): 1,25 (m, 8H), 2,85 (t, 2H), 3,30 (q, 2H), 3,35 (m, 4H), 3,40 (t, 2H), 3,60 (s, 4H), 3,80 (s, 3H), 4,18 (q, 4H), 6,80-7,05 (m, 4H, Ar.).

1.4.3. Kalium-N-[2-(N'-ethoxyethyl-N'-(2-methoxyphenyl)aminoethyl)]-imino-N,N-diacetat **(X3)**

Ein ähnliches Verfahren wie das oben in Abschnitt 1.1.5. beschriebene wurde verwendet, um **M16** zu hydrolysieren und eine Lösung von **X3** zu erhalten, welche für Absorptionsmessungen verwendet wurde.

1.5. Synthese von Kalium-N-[2-(N'-butyl-N'-(2-methoxyphenyl)aminoethyl)]-imino-N,N-diacetat (**X4**, siehe Figur 2B)

1.5.1. N-Butyl-2-anisidin (**M18**)

Eine Suspension von 6,2 g (50 mmol) o-Anisidin **M10**, 7,5 g (55 mmol) 1-Brombutan **M17,** 7,6 g (55 mmol) $K_2CO_3$ und 0,91 g (5,5 mmol) KI in 25 ml DMF wurde 24 Stunden auf 95 °C erhitzt. DMF wurde abgedampft, und der Rückstand wurde in 100 ml $CHCl_3$ und 100 ml gesättigtem NaCl gelöst. Die organische Phase wurde über $Na_2SO_4$ getrocknet. Das Lösungsmittel wurde abgedampft, wobei 9,4 g Rohöl erhalten wurden. Dieses Öl wurde mittels eines mit 45 g Silicagel 100 gepackten Plugs unter Verwendung von Cyclohexan als Eluierungsmittel gereinigt, wobei 4,8 g Reinprodukt erhalten wurden (Ausbeute: 55%).
$^1$H-NMR ($CDCl_3$), δ (ppm): 0,95 (t, 3H), 1,42 (m, 2H), 1,62 (m, 2H), 3,05 (m, 2H), 3,85 (s, 3H), 6,60-7,00 (m, 4H).

1.5.2. Diethyl-N-[2-(N'-butyl-N'-(2-methoxyphenyl)aminoethyl)]-imino-N,N-diacetat **(M19)**

Eine Suspension von 0,36 g (2 mmol) **M18,** 0,65 g (2,2 mmol) **M9** (Synthese siehe 1.3.1.) und 0,30 g (2,2 mmol) $K_2CO_3$ in 2 ml DMF wurde 18 Stunden auf 85 °C erhitzt. Das Gemisch wurde gekühlt und mit 25 ml $CHCl_3$ und 25 ml Wasser verdünnt. Die organische Phase wurde mit 25 ml gesättigtem NaCl gewaschen und über $Na_2SO_4$ ge-

trocknet. Das Lösungsmittel wurde abgedampft, wobei 1,05 g Rohprodukt erhalten wurden. Dieses Rohprodukt wurde mittels einer mit 5,5 g Silicagel gepackten Säule gereinigt, wobei zur Entfernung von nicht umgesetztem **M18** mit $CHCl_3$ und zur Gewinnung des Reinprodukts mit $CHCl_3$/Ethylacetat (4/1, v/v) eluiert wurde. Es wurden 0,31 g Reinprodukt erhalten (Ausbeute: 39%).
$^1$H-NMR ($CDCl_3$), δ (ppm): 0,92 (t, 3H), 1,20 (t, 6H), 1,25 (m, 2H), 1,40 (m, 2H), 2,80 (t, 2H), 3,05 (t, 2H), 3,20 (t, 2H), 3,55 (s, 4H), 3,80 (s, 3H), 4,18 (q, 4H), 6,80-7,05 (m, 4H, Ar.).

1.5.3. Kalium-N-[2-(N'-butyl-N'-(2-methoxyphenyl)aminoethyl)]-imino-N,N-diacetat **(X4)**

Ein ähnliches Verfahren wie das oben in Abschnitt 1.1.5. beschriebene wurde verwendet, um **M19** zu hydrolysieren und eine Lösung von **X4** zu erhalten, welche für Absorptionsmessungen verwendet wurde.

1.6. Synthese von Kalium-N-[2-(N'-phenoxyethyl-N'-(2-methoxyphenyl)aminoethyl)]-imino-N,N-diacetat (**X5**, siehe Figur 2B)

1.6.1. N-(2-Phenoxyethyl)-2-anisidin **(M21)**

Eine Suspension von 6,2 g (50 mmol) o-Anisidin **M10,** 12,1 g (60 mmol) β-Bromphenetol **M20** und 8,3 g (60 mmol) $K_2CO_3$ in 25 ml Acetonitril wurde 18 Stunden unter Rückfluß erhitzt. Das Gemisch wurde gekühlt, mit 100 ml $CHCl_3$ verdünnt und mit 100 ml Wasser und 100 ml gesättigtem NaCl gewaschen. Die organische Phase wurde über $Na_2SO_4$ getrocknet. Das Lösungsmittel wurde abgedampft, wobei 14,5 g Rohöl erhalten wurden. Dieses Öl wurde mittels eines mit 60 g Silicagel 100 gepackten Plugs unter Verwendung von Cyclohexan als Eluierungsmittel gereinigt, wobei 6,4 g Reinprodukt erhalten wurden (Ausbeute: 51%).
$^1$H-NMR ($CDCl_3$), δ (ppm): 3,45 (t, 2H), 3,85 (s, 3H), 4,05 (t, 2H), 6,80-7,20 (m, 9H).

1.6.2. Diethyl-N-[2-(N'-phenoxyethyl-N'-(2-methoxyphenyl)aminoethyl)]-imino-N,N-diacetat **(M22)**

Eine Suspension von 0,97 g (4 mmol) **M21**, 1,78 g (6 mmol) **M9** (Synthese siehe 1.3.1.) und 0,83 g (6 mmol) $K_2CO_3$ in 4 ml Acetonitril wurde 18 Stunden auf 85 °C erhitzt. Das Gemisch wurde gekühlt und mit 50 ml $CHCl_3$ und 50 ml Wasser verdünnt. Die organische Phase wurde mit 50 ml gesättigtem NaCl gewaschen und über $Na_2SO_4$ getrocknet. Das Lösungsmittel wurde abgedampft, wobei 2,60 g Rohprodukt erhalten wurden. Dieses Öl wurde mittels einer mit 13 g Silicagel gepackten Säule gereinigt, wobei zur Entfernung von nicht umgesetztem **M21** mit $CHCl_3$/Cyclohexan (1/1, v/v) und zur Gewinnung des Reinprodukts mit $CHCl_3$/Ethylacetat (4/1, v/v) eluiert wurde. Es wurden 1,20 g Reinprodukt erhalten (Ausbeute: 66%).
$^1$H-NMR ($CDCl_3$), δ (ppm): 1,25 (t, 6H), 2,85 (t, 2H), 3,20 (t, 2H), 3,45 (t, 2H), 3,60 (s, 4H), 3,80 (s, 3H), 4,00 (t, 2H), 4,18 (q, 4H), 6,80-7,05 (m, 4H, Ar.).

1.6.3. Kalium-N-[2-(N'-phenoxyethyl-N'-(2-methoxyphenyl)aminoethyl)]-imino-N,N-diacetat **(X5)**

Ein ähnliches Verfahren wie das oben in Abschnitt 1.1.5. beschriebene wurde verwendet, um **M22** zu hydrolysieren und eine Lösung von **X5** zu erhalten, welche für Absorptionsmessungen verwendet wurde.

1.7. Synthese von Kalium-o-anisidin-N,N-diethoxyacetat (**X6**, siehe Figur 2C)

1.7.1. 2-Chlorethoxyessigsäure **(M24)**

100 g (800 mmol) 2-Chlorethoxyethanol **M23** wurden bei 55°C langsam über 8 Stunden zu 500 ml konzentrierter $HNO_3$ (70%) hinzugefügt. Die Lösung wurde 18 Stunden bei Raumtemperatur gerührt und im siedenden Wasserbad 1 Stunde erhitzt, abgekühlt und in 500 ml Eiswasser gegossen. Die verdünnte Lösung wurde 8 x mit 1 1 $CHCl_3$ extrahiert. Alle Extrakte wurden vereinigt und abgedampft, wobei 54,2 g Öl erhalten wurden (Ausbeute: 49%). Dieses Öl wurde ohne weitere Reinigung direkt für die nächste Veresterung eingesetzt.

1.7.2. Ethyl-2-chlorethoxyacetat (**M25**)

54,1 g (390 mmol) des im vorhergehenden Reaktionsschritt erhaltenen **M24** wurden in 380 ml absolutem Ethanol gelöst und 9 ml konzentrierte $H_2SO_4$ hinzugefügt. Das Gemisch wurde 18 Stunden unter Rückfluß erhitzt. Der größte Teil des Ethanols wurde abgedampft, und der Rückstand wurde in 400 ml $CHCl_3$/100 ml Wasser gelöst und mit $NaHCO_3$-Pulver alkalisiert. Die organische Phase wurde mit 2x400 ml gesättigtem $NaHCO_3$ gewaschen und über

$Na_2SO_4$ getrocknet. Das Lösungsmittel wurde abgedampft, wobei 58,3 g klares Öl erhalten wurden (Ausbeute: 89%). $^1$H-NMR ($CDCl_3$), δ (ppm): 1,25 (t, 3H), 3,68 (t, 2H), 3,82 (t, 2H), 4,15 (s, 2H), 4,20 (q, 4H).

1.7.3. Diethyl-o-anisidin-N,N-diethoxyacetat **(M26)**

Eine Suspension von 0,37 g (3 mmol) o-Anisidin **M10,** 1,34 g (8 mmol) Ethyl-2-chlorethoxyacetat **M25,** 1,10 g (8 mmol) $K_2CO_3$ und 0,67 g (4 mmol) KI in 3 ml DMF wurde 7 Stunden auf 95°C erhitzt. Dünnschichtchromatographie zeigte, daß eine Menge monoalkyliertes Produkt vorlag. 1,34 g (8 mmol) zusätzliches Ethyl-2-chlorethoxyacetat **M25** und 1,10 g (8 mmol) zusätzliches $K_2CO_3$ wurden hinzugefügt. Das Erhitzen wurde für weitere 18 Stunden fortgesetzt. Das Gemisch wurde gekühlt und mit 50 ml Wasser/50 ml $CHCl_3$ verdünnt. Die organische Phase wurde mit 50 ml gesättigtem NaCl gewaschen und über $Na_2SO_4$ getrocknet. Das Lösungsmittel wurde abgedampft, wobei 1,08 g Rohöl erhalten wurden. Dieses Öl wurde mittels eines mit 5 g Silicagel 100 gepackten Plugs gereinigt, wobei als Eluierungsmittel Cyclohexan/$CHCl_3$ zur Entfernung von Vorlaufverunreinigungen

und dann $CHCl_3$/Ethylacetat (4/1, v/v) eingesetzt wurden, wobei 0,38 g hellgelbes Öl erhalten wurden (Ausbeute: 32%).
$^1$H-NMR ($CDCl_3$), δ (ppm): 1,22 (t, 6H), 3,42 (t, 3H), 3,60 (t, 3H), 3,80 (s, 3H), 4,02 (s, 4H), 4,15 (q, 4H), 6,80-7,05 (m, 4H).

1.7.4. Kalium-o-anisidin-N,N-diethoxyacetat **(X6)**

Ein ähnliches Verfahren wie das oben in Abschnitt 1.1.5. beschriebene wurde verwendet, um **M26** zu hydrolysieren und eine Lösung von **X6** zu erhalten, welche für Absorptionsmessungen verwendet wurde.

1.8. Synthese von Kalium-o-phenetidin-N,N-diethoxyacetat **(X7,** siehe Figur 2C)

1.8.1. Diethyl-o-phenetidin-N,N-diethoxyacetat (**M28**)

Eine Suspension von 0,68 g (5 mmol) o-Phenitidin **M27,** 2,50 g (15 mmol) Ethyl-2-chlorethoxyacetat **M25** (Synthese siehe 1.7.), 2,07 g (15 mmol) $K_2CO_3$ und 1,25 g (7,5 mmol) KI in 3 ml DMF wurde 20 Stunden auf 95°C erhitzt. Das Gemisch wurde gekühlt und mit 80 ml Wasser/80 ml $CHCl_3$ verdünnt. Die organische Phase wurde mit 80 ml gesättigtem NaCl gewaschen und über $Na_2SO_4$ getrocknet. Das Lösungsmittel wurde abgedampft, wobei 2,05 g Rohöl erhalten wurden. Dieses Öl wurde mittels eines mit 5 g Silicagel 100 gepackten Plugs gereinigt, wobei als Eluierungsmittel Cyclohexan/$CHCl_3$ zur Entfernung von Vorlaufverunreinigungen und dann $CHCl_3$/Ethylacetat (4/1, v/v) eingesetzt wurden. Es wurden 0,84 g hellgelbes Öl erhalten (Ausbeute: 42%).
$^1$H-NMR ($CDCl_3$), δ (ppm): 1,22 (t, 6H), 1,42 (t, 3H), 3,42 (t, 3H), 3,62 (t, 3H), 4,02 (q, 2H), 4,05 (s, 4H), 4,20 (q, 4H), 6,80-7,05 (m, 4H).

1.8.2. Kalium-o-phenetidin-N,N-diethoxyacetat (**X7**)

Ein ähnliches Verfahren wie das oben in Abschnitt 1.1.5. beschriebene wurde verwendet, um **M28** zu hydrolysieren und eine Lösung von **X7** zu erhalten, welche für Absorptionsmessungen verwendet wurde.

1.9. Synthese von Kalium-5-chlor-o-anisidin-N,N-diethoxyacetat (**X8**, siehe Figur 2C)

1.9.1. Diethyl-5-chlor-o-anisidin-N,N-diethoxyacetat (**M30**)

Eine Suspension von 0,78 g (5 mmol) 5-Chlor-2-methoxy-anilin **M29**, 2,50 g (15 mmol) Ethyl-2-chlorethoxyacetat **M25** (Synthese siehe 1.7.), 2,07 g (15 mmol) $K_2CO_3$ und 1,25 g (7,5 mmol) KI in 30 ml DMF wurde 18 Stunden auf 95°C erhitzt. DMF wurde abgedampft, und der Rückstand wurde mit 80 ml Wasser/80 ml $CHCl_3$ verdünnt. Die organische Phase wurde mit 80 ml gesättigtem NaCl gewaschen und über $Na_2SO_4$ getrocknet. Das Lösungsmittel wurde abgedampft, wobei 2,70 g Rohöl erhalten wurden. Dieses Öl wurde mittels eines mit 5 g Silicagel 100 gepackten Plugs gereinigt, wobei als Eluierungsmittel Cyclohexan/$CHCl_3$ zur Entfernung von Vorlaufverunreinigungen und dann $CHCl_3$/Ethylacetat (4/1, v/v) eingesetzt wurden. Es wurden 0,54 g hellgelbes Öl erhalten (Ausbeute: 26%).
$^1$H-NMR ($CDCl_3$), δ (ppm): 1,25 (t, 6H), 3,45 (t, 3H), 3,62 (t, 3H), 3,80 (s, 3H), 4,02 (s, 4H), 4,20 (q, 4H), 6,60-7,05 (m, 3H).

1.9.2. Kalium-5-chlor-o-anisidin-N,N-diethoxyacetat (**X8**)

Ein ähnliches Verfahren wie das oben in Abschnitt 1.1.5. beschriebene wurde verwendet, um **M30** zu hydrolysieren und eine Lösung von **X8** zu erhalten, welche für Absorptionsmessungen verwendet wurde.

2. Synthese von erfindungsgemäßen Luminophor-Ionophoren

Luminophor-Ionophore auf der Grundlage von Naphthalimid wurden nach zwei verschiedenen Verfahren hergestellt. Die Syntheseschemata sind in den Figuren 3A und 3B dargestellt.

Ein Luminophor-Ionophor, welcher **X3*** als Ionophor aufweist (entspricht **X3** in Fig. 1, außer daß in para-Stellung zum o-Anisidin-Sauerstoff H durch Methoxy ersetzt ist) wurde ausgehend von einer kommerziell erhältlichen Vorstufe, 2,5-Dimethoxyphenethylamin **J1,** synthetisiert (Figur 3A). Diese Verbindung wurde in einem $HNO_3$/HCl-Gemisch nitriert, gefolgt von BOC-Schutz (BOC = t-Butoxycarbonyl), Hydrogenierung, Monoalkylierung und Entschützung, und wurde dann an das 4-Chlornaphthalimidderivat **C3** gekoppelt, welches eine t-Butyl-geschützte Carbonsäuregruppe enthält. Der erhaltene Luminophor **J7** wurde mit **M9** (siehe Figur 2B) alkyliert, um den Triester **J8** zu ergeben. Der t-Butylester wurde mit Trifluoressigsäure (TFA) entfernt, und die freie Carboxylgruppe wurde für die Immobilisierung von **J9** auf Aminocellulose verwendet. Der Ethylester wurde in wäßriger KOH hydrolisiert, um das Endprodukt **J11** zu erhalten.

Nach demselben Syntheseschema wurde auch ein Luminophor-Ionophor hergestellt, welcher **X2*** (entspricht **X2** in Fig. 1, außer daß in para-Stellung zum o-Anisidin-Sauerstoff H durch Methoxy ersetzt ist) als Ionophor aufweist.

Ein anderer Luminophor-Ionophor, welcher **X7*** als Ionophor aufweist (entspricht **X7** in Fig. 1, außer daß in para-Stellung zum o-Anisidin-Sauerstoff H durch Ethoxy ersetzt ist), wurde aus 2,5 Dihydroxybenzaldehyd **N2** als Ausgangsmaterial hergestellt (Figur 3B), da das Ethylanalog von **J1** kommerziell nicht erhältlich war. **N2** wurde mit Iodethan alkyliert und mit Nitromethan kondensiert, um das Nitrostyrolderivat **N4** zu erhalten, welches zum Phenethylaminderivat **N5** reduziert wurde. **N5** wurde nitriert, hydrogeniert und an das 4-Chlornaphthalimidderivat **C3** gekoppelt. Das erhaltene Amin **N8** wurde mit **M25** (Synthese siehe 1.7. und Figur 2C) alkyliert, um den Triester **N9** zu erhalten, welcher entschützt, auf Aminocellulose immobilisiert und hydrolisiert wurde, um das Endprodukt **N12** zu erhalten.

2.1. Synthese eines Luminophor-Ionophors mit **X3*** als Ionophor

2.1.1. 4-Nitro-2,5-dimethoxyphenethylamine **(J2)**

45,5 g (250 mmol) 2,5-Dimethoxyphenethylamin **J1** wurde mit 40 ml Wasser gemischt und auf 0°C gekühlt, während 30 ml konzentrierte HCl langsam zugegeben wurden. Die erhaltene milchige Emulsion wurde auf 0 bis 5°C gekühlt, und 65 ml konzentrierte $HNO_3$ wurde langsam und vorsichtig innerhalb von etwa 2 Stunden hinzugefügt, wobei die Temperatur unter 10°C gehalten wurde. Das Gemisch erstarrte, als etwa die Hälfte der $HNO_3$ zugesetzt war. 50 ml Eiswasser wurden hinzugefügt, um das Gemisch rührbar zu machen. Dann wurde die restliche $HNO_3$ zugesetzt. Das Gemisch wurde auf Raumtemperatur erwärmt und 3 Stunden bei Raumtemperatur gerührt, mit 40%iger NaOH auf pH>12 alkalisiert und dann mit 2x11 $CHCl_3$ extrahiert. Der $CHCl_3$-Extrakt wurde mit 3x1 1 0,5 M NaOH rückgewaschen und über 15 g $K_2CO_3$ getrocknet. Das Lösungsmittel wurde abgedampft, wobei 49,8 g orangefarbenes Öl erhalten wurden (Ausbeute: 88%), welches beim Abkühlen auskristallisierte.
$^1$H-NMR ($CDCl_3$), δ (ppm): 1,30 (br.s, 2H), 2,80 (t, 2H), 2,95 (t, 2H), 3,80 (s, 3H), 3,92 (s, 3H), 6,90 (s, 1H), 7,20 (s, 1H).

2.1.2. N-t-Butoxycarbonyl-4-nitro-2,5-dimethoxyphenethylamin (**J3**)

Zu einer Lösung von 22,6 g (100 mmol) 4-Nitro-2,5-dimethoxyphenethylamin **J2** und 15,5 g (120 mmol) Triethylamin in 125 ml $CHCl_3$ wurden 26,2 g (120 mmol) Di-t-butyldicarbonat in 25 ml $CHCl_3$ hinzugefügt. Das Gemisch wurde 15 min bei Raumtemperatur gerührt, mit 3x200 ml 0,4 M HCl gewaschen und über $Na_2SO_4$ getrocknet. Das Lösungsmittel wurde abgedampft, wobei 36 g Öl erhalten wurden. Dieses Öl wurde in 20 ml Ethylacetat gelöst, dann wurden 200 ml Hexan zugegeben, der erhaltene Niederschlag wurde abfiltriert, mit 2x100 ml Hexan gewaschen und 18 Stunden bei Raumtemperatur getrocknet, wobei 27,4 g hellgelbes fasriges Pulver erhalten wurden (Ausbeute: 84%).
$^1$H-NMR ($CDCl_3$), δ (ppm): 1,40 (s, 9H), 2,82 (t, 2H), 3,35 (t, 2H), 3,80 (s, 3H), 3,90 (s, 3H), 6,90 (s, 1H), 7,20 (s, 1H).

2.1.3. N-t-Butoxycarbonyl-4-amino-2,5-dimethoxyphenethylamin **(J4)**

27 g (83 mmol) N-t-Butoxycarbonyl-4-nitro-2,5-dimethoxyphenethylamin **J3** wurden in 300 ml Methanol gelöst, und 1,5 g 10% Palladium auf Carbon-Black wurden hinzugefügt. Diese Suspension wurde 18 Stunden bei 2,2 atm. hydrogeniert, bis keine weitere Wasserstoffaufnahme mehr beobachtet wurde. Der Katalysator wurde abfiltriert,

und das Lösungsmittel wurde abgedampft, wobei 22,3 g weißes Pulver erhalten wurden (Ausbeute: 98%).
$^{1}$H-NMR ($CDCl_3$), δ (ppm): 1,40 (s, 9H), 1,55 (s, 2H), 2,65 (t, 2H), 3,25 (t, 2H), 3,70 (s, 3H), 3,80 (s, 3H), 6,30 (s, 1H), 6,58 (s, 1H).

2.1.4. N-t-Butoxycarbonyl-4-(N'-ethoxyethyl)amino-2,5-dimethoxyphenethylamin **(J5)**

Ein Gemisch von 11,9 g (40 mmol) **J4,** 5,2 g (48 mmol) Chlorethylethylether **M14** (siehe Figur 2B), 11,6 g (48 mmol) $K_2CO_3$, 4,0 g (24 mmol) KI und 20 ml DMF wurde 18 Stunden auf 95°C erhitzt. DMF wurde abgedampft, und der Rückstand wurde in 200 ml $CHCl_3$ und 200 ml Wasser gelöst. Die organische Phase wurde mit 200 ml gesättigtem NaCl gewaschen und über $Na_2SO_4$ getrocknet. Das Lösungsmittel wurde abgedampft, wobei 12 g Öl erhalten wurden. Dieses Öl wurde mittels eines mit 60 g Silicagel 100 gepackten Plugs unter Verwendung von Cyclohexan als Eluierungsmittel gereinigt, wobei 2,65 g Reinprodukt erhalten wurden (Ausbeute: 18%).
$^{1}$H-NMR ($CDCl_3$), δ (ppm): 1,25 (t, 3H), 1,40 (s, 9H), 1,55 (s, 2H), 2,65 (t, 2H), 3,30 (m, 4H), 3,55 (q, 2H), 3,65 (t, 2H), 3,70 (s, 3H), 3,80 (s, 3H), 4,45 (br.s, 1H), 4,70 (br.s, 1H), 6,25 (s, 1H), 6,55 (s, 1H).

2.1.5. 4-(N-ethoxyethyl)amino-2,5-dimethoxyphenethylamin (**J6**)

2,60 g (7,1 mmol) **J5** wurden in einem Gemisch von 10 ml Trifluoressigsäure und 1 ml Wasser gelöst. Das Gemisch wurde 15 min bei Raumtemperatur gerührt, mit 20 ml Wasser verdünnt, mit gesättigtem $K_2CO_3$ auf pH~12 alkalisiert und mit 2x40 ml $CHCl_3$ extrahiert. Der Extrakt wurde über $K_2CO_3$ getrocknet. Das Lösungsmittel wurde abgedampft, wobei 1,78 g Öl erhalten wurden (Ausbeute: 92%).
$^{1}$H-NMR ($CDCl_3$), δ (ppm): 1,25 (t, 3H), 1,60 (br. 2H), 2,65 (t, 2H), 2,90 (t, 3H), 3,30 (t, 2H), 2,90 (t, 3H), 3,30 (t, 2H), 3,55 (q, 2H), 3,70 (t, 2H), 3,80 (s, 3H), 3,85 (s, 3H), 4,45 (br.s, 1H), 6,30 (s, 1H), 6,60 (s, 1H).

2.1.6. 4-Chlor-1,8-naphthalimidylmethylbenzoesäure **(C2)**

46,4 g (200 mmol) 4-Chlor-1,8-naphthalanhydrid **C1** und 30,2 g (200 mmol) 4-Aminomethylbenzoesäure wurden in 1 1 DMF suspendiert, 16 Stunden bei Raumtemperatur und dann 6 Stunden bei 60°C gerührt. Das Gemisch wurde in 3 l Wasser gegossen und der pH mit 6 N HCl auf 4 eingestellt. Der erhaltene Niederschlag wurde abfiltriert und 18 Stunden bei 60°C getrocknet, wobei 36 g weißliches Pulver erhalten wurden (Ausbeute: 51%).
$^{1}$H-NMR ($CDCl_3$), δ (ppm): 5,30 (s, 2H), 7,45 (d, 2H), 7,85 (d, 2H), 8,02 (q, 2H), 8,45 (d, 1H), 8,60 (t, 2H).

2.1.7. t-Butyl-4-Chlor-1,8-naphthalimidylmethylbenzoat **(C3)**

Zu einer Suspension von 29,2 g (80 mmol) **C2** in 320 ml DMF, welche bei 40°C unter einem Stickstoffstrom gerührt wurde, wurden 52,0 g (320 mmol) 1,1'-Carbonyldiimidazol langsam über 20 min zugegeben. Die Suspension wurde zu einer klaren Lösung und wurde in 15 min wieder trüb. Dann wurde das Gemisch auf 70°C erhitzt und nach Zugabe von 52 ml (1600 mmol) t-Butanol und 48 ml (320 mmol) 1,8-Diazabicyclo[5,4,0]undec-7-en (DBU) 18 Stunden auf dieser Temperatur gehalten. Das Gemisch wurde gekühlt und unter heftigem Rühren in 2,0 1 eisige 1 N HCl gegossen. Der erhaltene Niederschlag wurde abfiltriert, mit 2x300 ml 1 N HCl gewaschen, und nach Trocknen in einem Exsikkator mit $P_2O_5$ für 18 Stunden wurden 28,5 g Rohprodukt erhalten. Diese Rohprodukt wurde mittels einer Silicagelsäule unter Verwendung von $CHCl_3$/Cyclohexan als Eluierungsmittel gereinigt, wobei 12,0 g weißes Pulver erhalten wurden (Ausbeute: 36%).
$^{1}$H-NMR ($CDCl_3$), δ (ppm): 1,50 (s, 9H), 5,30 (s, 2H), 7,45 (d, 2H), 7,80 (d, 2H), 8,05 (q, 2H), 8,50 (d, 1H), 8,60 (t, 2H).

2.1.8. t-Butyl-4-{4-[4-(N-ethoxyethylamino)-2,5-dimethoxyphenylethylamino]-1,8-naphthalimidylmethyl}-benzoat **(J7)**

Eine Suspension von 1,70 g (6,3 mmol) **J6** und 0,88 g (2,1 mmol) **C3** in 2,2 ml N-Methylpyrrolidinon (NMP) wurde 18 Stunden auf 85°C erhitzt. Das Gemisch wurde gekühlt und in 45 ml Wasser gegossen. Der erhaltene Niederschlag wurde abfiltriert und mit 3x20 ml Wasser gewaschen und 18 Stunden über $P_2O_5$ getrocknet, wobei 1,2 g Rohprodukt erhalten wurden. Das Rohprodukt wurde mittels einer Silicagelsäule unter Verwendung von $CHCl_3$ als Eluierungsmittel gereinigt, wobei 0,92 g Reinprodukt erhalten wurden (Ausbeute: 67%):
$^{1}$H-NMR ($CDCl_3$), δ (ppm): 1,20 (t, 3H), 1,55 (s, 9H), 3,05 (t, 2H), 3,35 (t, 2H), 3,55 (m, 4H), 3,70 (t, 2H), 3,80 (s, 3H), 3,95 (s, 3H), 5,40 (s, 2H), 6,65 (t, 2H), 6,80 (t, 1H), 7,58 (m, 3H), 7,90 (d, 2H), 8,00 (d, 1H), 8,45 (d, 1H), 8,60 (d, 1H).

2.1.9. t-Butyl-4-{4-[4-(N-ethoxyethylamino)-4-(bis-ethoxycarbonylmethylaminoethyl)-2,5-dimethoxyphenylethylamino]-1,8-naphthalimidylmethyl}-benzoat **(J8)**

Eine Suspension von 0,90 g (1,38 mmol) **J7,** 1,23 g (4,41 mmol) **M9** (siehe Figur 2B) und 0,57 g (4,41 mmol) Diisopropylethylamin in 5,5 ml DMF wurden unter Stickstoffatmosphäre (Stickstoffballon) 20 Stunden auf 90°C erhitzt. Dann wurden weitere 0,67 g (2,20 mmol) **M9** zugesetzt. Das Gemisch wurde für weitere 18 Stunden erhitzt. DMF wurde abgedampft, und der Rückstand wurde in 50 ml $CHCl_3$ und 50 ml Wasser gelöst. Die organische Phase wurde mit 50 ml gesättigtem NaCl gewaschen und über $Na_2SO_4$ getrocknet. Das Lösungsmittel wurde abgedampft, wobei 2,7 g braunes Öl erhalten wurden. Dieses Öl wurde auf 13 g Silicagel 100 gereinigt, wobei als Eluierungsmittel $CHCl_3$ zur Entfernung von nicht umgesetztem **J7** und dann $CHCl_3$/Ethylacetat (4/1, v/v) eingesetzt wurden, wobei 0,40 g des gewünschten Reinproduktes erhalten wurden (Ausbeute: 32%).
$^1$H-NMR ($CDCl_3$), δ (ppm): 1,25 (m, 9H), 1,55 (s, 9H), 2,85 (t, 2H), 3,05 (t, 2H), 3,30 (s, 3H), 3,35 (t, 2H), 3,55 (m, 6H), 3,70 (t, 2H), 3,80 (s, 3H), 3,95 (s, 3H), 5,40 (s, 2H), 6,65 (t, 2H), 6,80 (t, 1H), 7,58 (m, 3H), 7,90 (d, 2H), 8,00 (d, 1H), 8,45 (d, 1H), 8,60 (d, 1H).

FABMS (70 eV, m-Nitrobenzylalkohol-Dispersion mit LiI): 876 (77%), (M+Li); 710 (100%), (dealkylierter Luminophor-Ionophor).

Berechnet für $C_{42}H_{49}N_3O_{13}$: C 66,36; H 6,96; N 6,45; gefunden: C 65,68; H 7,08; N 6,35.

2.1.10.4- {4-[4-(N-ethoxyethylamino)-4-(bis-ethoxycarbonylmethylaminoethyl)-2,5-dimethoxyphenylethylamino]-1,8-naphthalimidylmethyl}-benzoesäure **(J9)**

2 ml Trifluoressigsäure (TFA) wurden einer Lösung von 0,40 g (0,46 mmol) **J8** in 8 ml $CH_2Cl_2$ zugesetzt. Die erhaltene Lösung wurde etwa 1 Stunde bei Raumtemperatur gerührt, bis die Dünnschichtchromatographie anzeigte, daß **J8** größtenteils hydrolisiert war. Das Gemisch wurde mit 20 ml $CHCl_3$ verdünnt und abgedampft. Der Rückstand wurde in 20 ml $CHCl_3$ gelöst und wieder abgedampft. Dieser Vorgang wurde weitere zweimal wiederholt, um TFA vollständig zu entfernen, wonach 0,36 g gummiartige Substanz erhalten wurden (Ausbeute: 95%). Diese wurde direkt für die Immobilisierung verwendet.

2.1.11. Immobilisierung von 4-{4-[4-(N-ethoxyethylamino)-4-(bis-ethoxycarbonylmethylaminoethyl)-2,5-dimethoxyphenylethylamino]-1,8-naphthalimidylmethyl}-benzoesäure auf Aminocellulose (**J10**)

0,36 g (0,45 mmol) des Indikators **J9,** 0,93 g (4,5 mmol) N,N-Dicyclohexyl-1,3-carbodiimid, 0,52 g (4,5 mmol) N-Hydroxysuccinimid und 10 g (~ 3 meq.) aktivierte Cellulose (hergestellt gemäß SU-A - 1 028 677, CA 99:177723h) wurden 20 Stunden in 50 ml DMF suspendiert. Die Cellulosefaser wurde abfiltriert und mit 5x50 ml DMF, 50 ml Wasser, 2x50 ml 0,2 N HCl, 50 ml Wasser, 2x50 ml 0,2 N NaOH und 10x50 ml Wasser gewaschen. Die erhaltene Faser war für die Hydrolyse einsatzbereit.

2.1.12. Hydrolyse von auf Aminocellulose immobilisiertem 4-{4-[4-(N-ethoxyethylamino)-4-(bis-ethoxycarbonylmethylaminoethyl)-2,5-dimethoxyphenylethylamino]-1,8-naphthalimidylmethyl}-benzoesäure zur Freisetzung der Carboxylgruppen für die Calciumbindung **(J11)**

Das im vorhergehenden Schritt hergestellte Cellulosepulver mit immobilisiertem Indikator **(J10)** wurde in 50 ml 1 N KOH suspendiert. Die Suspension wurde 5 min auf 80 °C erhitzt, 3 Stunden bei Raumtemperatur gerührt, filtriert und mit 20x50 ml Wasser gewaschen, bis das Filtrat neutral war. Dann wurde mit 2x50 ml Aceton und 2x50 ml Ether gewaschen und vor dem Testen 16 Stunden bei Raumtemperatur getrocknet.

2.2. Synthese eines Luminophor-Ionophors mit **X7*** als Ionophor

2.2.1. 2,5-Diethoxybenzaldehyd **(N3)**

Eine Suspension von 9,66 g (70 mmol) 2,5-Dihydroxybenzaldehyd N2, 32,75 g (210 mmol) Iodethan, 24,19 g (175 mmol) $K_2CO_3$ in 350 ml Aceton wurde unter Stickstoff 18 Stunden unter Rückfluß erhitzt. Das Lösungsmittel wurde abgedampft, und der Rückstand wurde in 300 ml $CHCl_3$ und 300 ml Wasser gelöst. Die organische Phase wurde mit 2x300 ml 2,5%igem $Na_2CO_3$ und 300 ml gesättigtem NaCl gewaschen und über $Na_2SO_4$ getrocknet. Das Lösungsmittel wurde abgedampft und der Rückstand (13,5 g) wurde mittels einer Silicagelsäule (Silicagel 100, $CHCl_3$/Cyclohexan: 1/1, v/v) gereinigt, wobei 8,61 g hellgelbes Öl, welches nach Abkühlen auf Raumtemperatur auskristallisierte, erhalten wurden (Ausbeute: 63%).
$^1$H-NMR ($CDCl_3$), δ (ppm): 1,38 (t, 3H), 1,42 (t, 3H), 4,00 (q, 2H), 4,10 (q, 2H), 6,90 (d, 1H), 7,10 (d, 1H), 7,30 (s, 1H),

10,45 (s, 1H).

2.2.2. 2,5-Diethoxy-β-nitrostyrol (**N4**)

Ein Gemisch von 8,35 g (43 mmol) **N3**, 26,24 g (430 mmol) Nitromethan, 33,10 g (430 mmol) Ammoniumacetat in 86 ml Essigsäure wurde langsam auf 80°C erhitzt und 2 Stunden auf dieser Temperatur gehalten. Nach Abkühlung auf Raumtemperatur, wurde das Gemisch in 800 ml Eiswasser gegossen. Der erhaltene Niederschlag wurde abfiltriert, mit 3x100 ml Wasser gewaschen und 18 Stunden über $P_2O_5$ getrocknet, wobei 8,15 g gelbe Prismen erhalten wurden (Ausbeute: 80%).
$^1$H-NMR ($CDCl_3$), δ (ppm): 1,40 (t, 3H), 1,50 (t, 3H), 4,00 (q, 2H), 4,12 (q, 2H), 6,85 (d, 1H), 6,96 (m, 2H), 7,84 (d, 1H), 8,60 (d, 1H).

2.2.3. 2,5-Diethoxyphenethylamin **(N5)**

Eine Lösung von 8,00 g (33,7 mmol) **N4** in 50 ml THF wurde langsam über 1 Stunde einer Suspension von 13,37 g (337 mmol) Lithiumaluminiumhydrid in 500 ml THF auf Siedetemperatur zugesetzt. Das Gemisch wurde für weitere 4 Stunden unter Rückfluß erhitzt. Das Gemisch wurde dann in einem Eiswasserbad auf ~ 15°C abgekühlt und mit 18%iger NaOH gelöscht. Der Niederschlag wurde abfiltriert, das Filtrat bis zur Trockne eingedampft und der Rückstand in 50 ml $CHCl_3$ gelöst. Diese Lösung wurde mit 2x50 ml 1 N HCl extrahiert. Die wäßrigen Extrakte wurden mit 40%iger NaOH auf pH>12 alkalisiert, dann mit 2x50 ml $CHCl_3$ extrahiert und über $K_2CO_3$ getrocknet. Das Lösungsmittel wurde abgedampft, wobei 5,80 g hellgelbes Öl erhalten wurden (Ausbeute: 84%).
$^1$H-NMR ($CDCl_3$), δ (ppm): 1,40 (t, 6H), 1,80 (br.s, 2H), 2,75 (t, 2H), 2,95 (t, 2H), 4,00 (q, 4H), 6,75 (m, 3H).

2.2.4. 4-Nitro-2,5-diethoxyphenethylamin **(N6)**

5,23 g (25 mmol) 2,5-Diethoxyphenethylamin **N5** wurden mit 10 ml Wasser gemischt und auf 0°C gekühlt, während 3 ml konzentrierte HCl langsam zugesetzt wurden. Die erhaltene milchige Emulsion wurde auf 0 bis 5°C gekühlt, und 13,5 ml konzentrierte $HNO_3$ wurden langsam und vorsichtig innerhalb von etwa 2 Stunden zugesetzt. Die Temperatur wurde unter 10°C gehalten. Das Gemisch erstarrte, als etwa die Hälfte der $HNO_3$ zugesetzt war. 50 ml Eiswasser wurde hinzugefügt, um das Gemisch rührbar zu machen. Dann wurde die restliche $HNO_3$ zugesetzt. Das Gemisch wurde auf Raumtemperatur erwärmt und 3 Stunden bei Raumtemperatur gerührt, mit 40%iger NaOH auf pH>12 alkalisiert und dann mit 2x100 ml $CHCl_3$ extrahiert. Der $CHCl_3$-Extrakt wurde mit 3x100 ml 0,5 M NaOH rückgewaschen und über 10 g $K_2CO_3$ getrocknet. Das Lösungsmittel wurde abgedampft, wobei 3,22 g orangefarbenes Öl erhalten wurden (Ausbeute: 52%), welches nach Abkühlen auskristallisierte.
$^1$H-NMR($CDCl_3$), δ (ppm): 1,40 (m, 6H), 1,75 (br.s, 2H), 2,80 (t, 2H), 2,95 (t, 2H), 4,05 (q, 2H), 4,15 (q, 2H), 6,90 (s, 1H), 7,40 (s, 1H).

2.2.5. 4-Amino-2,5-diethoxyphenethylamin **(N7)**

3,05 g (12,0 mmol) 4-Nitro-2,5-diethoxyphenethylamin **N6** wurden in 50 ml Ethanol gelöst, und 1,5 g 10% Palladium auf Carbon-Black wurden hinzugefügt. Diese Suspension wurde 18 Stunden bei 2,2 atm. hydrogeniert, bis keine weitere Wasserstoffaufnahme mehr beobachtet wurde. Der Katalysator wurde abfiltriert, und das Lösungsmittel wurde abgedampft, wobei 2,75 g hellgelbes Öl erhalten wurden (Ausbeute: 102%).
$^1$H-NMR ($CDCl_3$), δ (ppm): 1,25 (m, 6H), 1,40 (br.t, 4H), 2,90 (t, 2H), 3,20 (t, 2H), 3,75 (q, 2H), 3,95 (m, 2H), 6,30 (s, 1H), 6,65 (s, 1H).

2.2.6. t-Butyl-4-[4-(4-amino-2,5-diethoxyphenylethylamino)-1,8-naphthalimidylmethyl]-benzoat **(N8)**

Eine Suspension von 2,70 g (12,0 mmol) **N7** und 1,68 g (4 mmol) **C3** (siehe 2.1.7. und Figur 3A) in 6 ml N-Methylpyrrolidinon (NMP) wurde 5 Stunden auf 85°C erhitzt. Das Gemisch wurde abgekühlt und in 114 ml Wasser gegossen. Der erhaltene Niederschlag wurde abfiltriert, mit 3x20 ml Wasser gewaschen und 18 Stunden über $P_2O_5$ getrocknet, wobei 1,9 g Rohprodukt erhalten wurden. Das Rohprodukt wurde mittels einer Silicagelsäule unter Verwendung von $CHCl_3$ als Eluierungsmittel gereinigt, wobei 0,47 g Reinprodukt erhalten wurden (Ausbeute: 19%).
$^1$H-NMR ($CDCl_3$), δ (ppm): 1,35 (t, 3H), 1,45 (t, 3H), 1,55 (s, 9H), 3,10 (t, 2H), 3,60 (t, 2H), 3,98 (q, 2H), 4,12 (q, 2H), 5,40 (s, 2H), 6,75 (m, 1H), 7,58 (m, 3H), 7,90 (d, 2H), 8,00 (d, 1H), 8,45 (d, 1H), 8,60 (d, 1H).

2.2.7. t-Butyl-4-{4-[4-(bis-ethoxycarbonylmethoxyethylamino)-2,5-diethoxyphenylethylamino]-1,8-naphthalimidylmethyl}-benzoat **(N9)**

Eine Suspension von 0,46 g (0,75 mmol) **N8,** 0,50 g (3 mmol) **M25** (Synthese siehe 1.7. und Figur 2C), 0,42 g (3 mmol) $K_2CO_3$ und 0,25 g (1,5 mmol) KI in 5 ml DMF wurde unter Stickstoffatmosphäre (Stickstoffballon) 20 Stunden auf 83°C erhitzt. Dann wurden weitere 0,50 g (2,20 mmol) **M25** und weitere 0,42 g $K_2CO_3$ zugesetzt. Das Gemisch wurde weitere 13 Stunden erhitzt. DMF wurde abgedampft, und der Rückstand wurde in 50 ml $CHCl_3$ und 50 ml Wasser gelöst. Die organische Phase wurde mit 50 ml gesättigtem NaCl gewaschen und über $Na_2SO_4$ getrocknet. Das Lösungsmittel wurde abgedampft, wobei 1,27 g braunes Öl erhalten wurden. Dieses Öl wurde auf 13 g Silicagel 100 gereinigt, wobei als Eluierungsmittel $CHCl_3$ zur Entfernung von nicht umgesetztem **N8** und dann $CHCl_3$/Ethylacetat (4/1, v/v) eingesetzt wurden. Es wurden 0,23 g des gewünschten Reinproduktes erhalten (Ausbeute: 35%).
$^1$H-NMR ($CDCl_3$), $\delta$ (ppm): 1,25 (m, 9H), 1,55 (s, 9H), 2,85 (t, 2H), 3,05 (t, 2H), 3,30 (s, 3H), 3,35 (t, 2H), 3,55 (m, 6H), 3,70 (t, 2H), 3,80 (s, 3H), 3,95 (s, 3H), 5,40 (s, 2H), 6,65 (t, 2H), 6,80 (t, 1H), 7,58 (m, 3H), 7,90 (d, 2H), 8,00 (d, 1H), 8,45 (d, 1H), 8,60 (d, 1H).

2.2.8. 4-{4-[4-(Bis-ethoxycarbonylmethoxyethylamino)-2,5-diethoxyphenylethylamino]-1,8-naphthalimidylmethyl}-benzoesäure (**N10**)

1 ml Trifluoressigsäure (TFA) wurde einer Lösung von 0,20 g (0,23 mmol) **N9** in 4 ml $CH_2Cl_2$ zugesetzt. Die erhaltene Lösung wurde etwa 1 Stunde bei Raumtemperatur gerührt, bis die Dünnschichtchromatographie anzeigte, daß **N9** größtenteils hydrolisiert war. Das Gemisch wurde mit 20 ml $CHCl_3$ verdünnt und abgedampft. Der Rückstand wurde in 20 ml $CHCl_3$ gelöst und wieder abgedampft. Dieser Vorgang wurde weitere zweimal wiederholt, um TFA vollständig zu entfernen, wonach 0,18 g gummiartige Substanz erhalten wurden (Ausbeute: 95%). Diese wurde direkt für die Immobilisierung verwendet.

2.2.9. Immobilisierung von 4-{4-[4-(Bis-ethoxycarbonylmethoxyethylamino)-2,5-diethoxyphenylethylamino]-1,8-naphthalimidylmethyl}-benzoesäure auf Aminocellulose **(N11)**

0,18 g (0,23 mmol) von **N10,** 0,46 g (2,3 mmol) N,N-Dicyclohexyl-1,3-carbodiimid, 0,26 g (2,3 mmol) N-Hydroxysuccinimid und 5 g (~ 1,5 meq.) aktivierte Cellulose (hergestellt gemäß SU-A - 1 028 677, CA 99:177723h) wurden 20 Stunden in 50 ml DMF suspendiert. Die Cellulosefaser wurde abfiltriert und mit 5x50 ml DMF, 50 ml Wasser, 2x50 ml 0,2 N HCl, 50 ml Wasser, 2x50 ml 0,2 N NaOH und 10x50 ml Wasser gewaschen. Die erhaltene Faser war für die Hydrolyse einsatzbereit.

2.2.10. Hydrolyse von auf Aminocellulose immobilisierter 4-{4-[4-(Bis-ethoxycarbonylmethoxyethylamino)-2,5-diethoxyphenylethylamino]-1,8-naphthalimidylmethyl}-benzoesäure zur Freisetzung der Carboxylgruppen für die Calciumbindung (**N12**)

Das im vorhergehenden Schritt hergestellte Cellulosepulver mit immobilisiertem Indikator (**N11**) wurde in 50 ml 1 N KOH suspendiert. Die Suspension wurde 5 min auf 80 °C erhitzt, 3 Stunden bei Raumtemperatur gerührt, filtriert und der Niederschlag mit 20x50 ml Wasser gewaschen, bis das Filtrat neutral war. Dann wurde mit 2x50 ml Aceton und 2x50 ml Ether gewaschen und vor dem Testen 16 Stunden bei Raumtemperatur getrocknet.

3. Herstellung erfindungsgemäßer optischer Sensoren (Sensorscheiben)

0,5 g gesiebte (25 μm) Aminocellulosefasem mit immobilisiertem Indikator **J11 (X3*),** welche wie in Abschnitt 2.1.12 beschrieben hergestellt worden waren, wurden in 9,5 g 10% Hydrogel D4 (Tyndale Plains-Hunter LTD. Ringoes, NJ 08551) in 90% Ethanol-Wasser 16 Stunden suspendiert. Die erhaltene homogene Dispersion wurde auf eine Polyesterfolie (Melinex Folie, ICI America) mit einer Endtrockenstärke von 10 um aufgetragen. Diese Folie wurde mit 3% Carbon-Black in 10% D4-Hydrogel in 90% Ethanol-Wasser mit einer Endtrockenstärke von 5 um überschichtet. Dann wurde eine kleine Scheibe mit einem Durchmesser von 2,5 cm herausgeschnitten und zur Aktivierung mindestens 16 Stunden in Puffer weichen gelassen.

Nach dem gleichen Verfahren wurden auch Sensorscheiben hergestellt, welche immobilisierte Indikatoren mit **X2*** bzw. **X7*** (=**N12**) als ionophore Gruppe enthielten.

4. Bestimmung der $K_d$-Werte erfindungsgemäßer Ionophore

4.1. Bestimmung der Absorptionseigenschaften erfindungsgemäßer Ionophore

Figur 4 zeigt die relativen Absorptionswerte ($Ca^{2+}$-Titrationskurven) der in Figur 1 dargestellten erfindungsgemäßen Ionophore **X1** bis **X9** in wäßriger Lösung (30 mmol/l Tris/HCl-Puffer; $CO_2$-frei; pH 7,4) in Abhängigkeit von der Konzentration an $Ca^{2+}$ (0,00001 bis 0,5 mol/l $Ca^{2+}$) bei einer Absorptionswellenlänge von 248 nm.

Die Absorptionswerte in Abhängigkeit von der $Ca^{2+}$-Konzentration wurden mittels eines handelsüblichen Photometers bei einer Absorptionswellenlänge von 248 nm gemessen. Die resultierenden Titrationskurven wurden bei einer $Ca^{2+}$-Konzentration von 0,00001 mol/l mittels Gleichung 4 (siehe unten) auf den Wert $A_{max}$ = 1 normiert.

4.2 Ermittlung der $K_d$-Werte der Ionophore

Die $K_d$-Werte der Ionophore wurden aus den gemessenen Absorptionswerten der $Ca^{2+}$-Titrationskurven nach Gleichung 3 (siehe oben) und Gleichung 4

$$(4) \qquad A_x = A_{max}\left(1 + \frac{Q - 1}{1 - 10^{(pKd - \log(cCa))}}\right)$$

ermittelt, wobei $A_x$ der normierte Absorptionswert bei gegebener $Ca^{2+}$-Konzentration ist, $A_{max}$ der Absorptionswert bei 0,00001 mol/l $Ca^{2+}$ ist und $pK_d$ die in Gleichung 3 angegebene Bedeutung besitzt. Der Parameter Q trägt der Tatsache Rechnung, daß die Absorptionswerte bei hohen $Ca^{2+}$-Konzentrationen nicht gegen Null gehen.

Die $K_d$-Werte für die erfindungsgemäßen Ionophore **X1** bis **X9** sind in Tabelle 1 angegeben. Sie wurden aus den gefundenen $pK_d$-Werten mittels Gleichung 3 ermittelt.

Tabelle 1

| Ionophor | $K_d$ (mol/l) | Gruppe[1)] |
|---|---|---|
| X1 | 0,123 | I |
| X2 | 0,00033 | I |
| X3 | 0,00104 | I |
| X4 | 0,00914 | I |
| X5 | 0,00696 | I |
| X6 | 0,00020 | II |
| X7 | 0,00109 | II |
| X8 | 0,00072 | II |
| X9 | 0,00616 | III |

1) Ionophore der Gruppe I weisen eine Gruppe mit der allgemeinen Formel II auf.
Ionophore der Gruppe II weisen eine Gruppe mit der allgemeinen Formel III auf.
Ionophore der Gruppe III weisen eine Gruppe mit der allgemeinen Formel IV auf.

- Gruppe I:

Bei den erfindungsgemäßen Ionophoren der Gruppe I sind die Imino-N,N-diacetat-Liganden mittels einer $-CH_2-CY-$ Gruppe (Y = $H_2$ oder O) am Stickstoffatom des o-Alkoxyanilins gebunden.

Durch Auswahl geeigneter Substituenten $R_1$ am Stickstoffatom bzw. $R_2$ am Sauerstoffatom bzw. $R_3$ am aromatischen Ring des o-Anisidins kann der $K_d$-Wert des Ionophors eingestellt werden. Für Ionophore mit Y = $H_2$ lassen sich beispielsweise $K_d$-Werte von 0,1-10 mmol/l einstellen (siehe Tabelle 1: **X2** bis **X5).** Verbindungen mit Y = O sind zur Bestimmung besonders hoher $Ca^{2+}$-Konzentrationen (> 10 mmol/l) geeignet (siehe Tabelle 1: **X1).**

Geeignete Substituenten $R_1$ sind beispielsweise Methoxyethyl (**X1** und **X2**), Ethoxyethyl **(X3),** Phenoxyethyl **(X5)** oder n-Butyl (**X4**). Geeignete Substituenten $R_2$ sind beispielsweise Methyl **(X2** bis **X5),** Ethyl oder Propyl. Geeignete Substituenten $R_3$ am aromatischen Ring sind elektronenziehende oder elektronengebende (beispielsweise H, **X1** bis **X5)** Gruppen.

- Gruppe II:

Die erfindungsgemäßen Ionophore der Gruppe II enthalten Alkyloxyacetat-Gruppen ($-(CH_2)_n-O-CH_2-COOH$) im Ligandenteil, die am Stickstoffatom des o-Alkoxyanilins gebunden sind. n ist vorzugsweise 2, beispielsweise in den Verbindungen **X6** bis **X8**. Wie bei den Ionophoren der Gruppe I lassen sich die Kd-Werte durch Variation der Substituenten $R_4$ am Sauerstoffatom (vergleiche beispielsweise die $K_d$-Werte für **X6** und **X7** in Tabelle 1) bzw. durch das Einfügen elektronenziehender oder elektronengebender Gruppen $R_5$ am aromatischen Ring des o-Alkoxyanilins einstellen.

Geeignete Substituenten $R_4$ sind beispielsweise Methyl (**X6** und **X8**) oder Ethyl **(X7).** Als elektronengebende Gruppe ist beispielsweise H (**X6**, **X7**), als elektronenziehende Gruppe $R_5$ beispielsweise Cl geeignet (**X8**).

- Gruppe III:

Bei den erfindungsgemäßen Ionophoren der Gruppe III ist der Imino-N,N-diacetat-Ligandenteil mittels einer $-CH_2-CH_2-$ Gruppe am Sauerstoffatom des o-Alkoxyanilins gebunden. Das Stickstoffatom trägt zwei Alkoxyethyl- oder Phenoxyethylgruppen. Auch bei dieser Gruppe von Verbindungen können durch Variation der Reste die $K_d$-Werte eingestellt werden. Als Substituent $R_6$ ist beispielsweise Methyl geeignet (**X9**). Geeignete Substituenten $R_7$ sind elektronenziehende oder elektronengebende (beispielsweise H in **X9)** Gruppen.

5. Bestimmung der Lumineszenzeigenschaften erfindungsgemäßer Luminophor-Ionophore

Zur Messung der Lumineszenzintensität einiger erfindungsgemäßer Verbindungen (Luminophor-Ionophore) wurden gemäß oben beschriebenem Verfahren (siehe Abschnitt 3.) hergestellte Sensorscheiben in eine lichtdurchlässige thermostatisierte Meßzelle eingebracht und mit Proben P (siehe Figur 5), die unterschiedliche $Ca^{2+}$-Konzentrationen und unterschiedliche pH-Werte aufwiesen, in Kontakt gebracht.

Die Meßanordnung ist in Figur 5 schematisch dargestellt, wobei ein Abschnitt der Sensorscheibe mit S bezeichnet ist. Die im hydrophilen ionenpermeablen Polymer (Hydrogel) suspendierte, auf Aminocellulose immobilisierte erfindungsgemäße Verbindung ist mit I bezeichnet. Diese Schicht M wird von einem für die Anregungs- und Meßstrahlung durchlässigen Träger T, der eine transparente Folie ist, getragen.

Die optische Meßeinrichtung bestand aus einer blauen LED als Lichtquelle L, einer Photodiode M als Detektor, optischen Filtern A und F zur Auswahl der Wellenlängen, einer faseroptischen Anordnung zur Leitung des Anregungslichtes in die Polymerschicht M und zur Leitung des Emissionslichtes zum Photodetektor M sowie einer Einrichtung zur elektromagnetischen Signalverarbeitung (nicht dargestellt). Anregungsseitig wurde ein Interferenzfilter (Peak-Transmission bei 480 nm) und emissionsseitig ein 520 nm cut-off Filter verwendet.

Die Figuren 6 und 7 zeigen die Lumineszenzeigenschaften einiger erfindungsgemäßer Verbindungen, immobilisiert auf Cellulose, in Abhängigkeit von der Konzentration an $Ca^{2+}$ bzw. vom pH-Wert. Die Ordinaten der dargestellten Diagramme geben jeweils die relativen Lumineszenzintensitäten an.

- Figur 6:

Figur 6 zeigt die relative Lumineszenzintensität dreier erfindungsgemäßer, auf Aminocellulose immobilisierter Luminophor-Ionophore **(L-X2*, L-X3*=J11, L-X7*=N12)** in Abhängigkeit vom negativen dekadischen Logarithmus der Calciumkonzentration (0,0002, 0,0004, 0,0006, 0,0009, 0,0011, 0,0013, 0,0015, 0,0017, 0,0020, 0,0022, 0,0024 mol/l).

Als Meßmedien wurden 0,1 M HEPES-Puffer, $CO_2$-frei, pH 7,4 (37°C) mit unterschiedlichen Konzentrationen an $CaCl_2$ verwendet.

- Figur 7:

Figur 7 zeigt die relative Lumineszenzintensität zweier erfindungsgemäßer, auf Aminocellulose immobilisierter Luminophor-Ionophore **(L-X3*=J11, L-X7*=N12)** in Abhängigkeit vom pH-Wert (6,841, 6,932, 7,030, 7,149, 7,271, 7,396, 7,507, 7,603, 7,700).

Als Meßmedien wurden 0,1 M HEPES-Puffer mit unterschiedlichen Konzentrationen an HEPES-Säure und HEPES-Na-Salz und einer $CaCl_2$-Konzentration von 0,0013 mol/l verwendet.

Wie aus Figur 7 ersichtlich, weisen erfindungsgemäße Ionophore bzw. Luminophor-Ionophore mit am Stickstoff des o-Anisidins gebundenem Ethyl-imino-N,N-diacetat-Ligandenteil (Gruppe 1, Y = $H_2$) eine pH-Abhängigkeit bei physiologischen pH-Werten auf. Sie sind daher für jene Meßsituationen geeignet, bei denen der pH-Wert der Probe bekannt ist bzw. auf einen bekannten Wert eingestellt werden kann (z.B. durch pH-Puffer).

Aus Figur 7 ist weiters zu ersehen, daß erfindungsgemäße Ionophore bzw. Luminophor-Ionophore mit am

Stickstoff des o-Anisidins gebundenem Diethoxyacetat-Ligandenteil aufgrund ihrer nicht signifikanten pH-Abhängigkeit im physiologischen pH-Meßbereich besonders gut zur Bestimmung von $Ca^{2+}$ bei physiologischen pH-Werten geeignet sind.

## Patentansprüche

**1.** Verbindung mit der allgemeinen Formel I

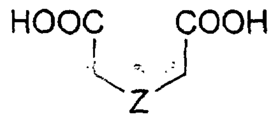

(I)

einschließlich deren Salze, worin Z entweder die Gruppe mit der allgemeinen Formel II

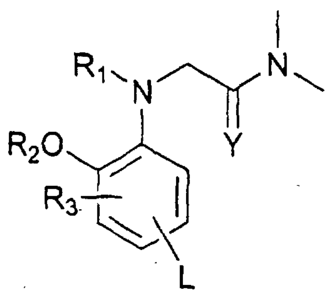

(II)

ist, in welcher

- $R_1$ Alkyl mit 1-4 C-Atomen, Alkoxyalkyl mit 2-5 C-Atomen oder Aryloxyalkyl, dessen Alkylrest 1-4 C-Atome aufweist, ist,
- $R_2$ Alkyl mit 1-4 C-Atomen oder Alkoxyalkyl mit 2-5 C-Atomen ist,
- $R_3$ H, Alkoxy mit 1-4 C-Atomen, Halogen, NO oder $NO_2$ ist,
- Y $H_2$ oder O ist und
- L ein luminophorer Rest in para- oder meta-Stellung zum Stickstoff ist,

oder die Gruppe mit der allgemeinen Formel III

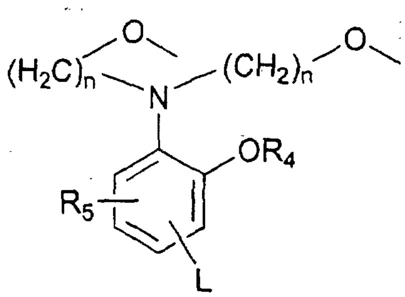

(III)

ist, in welcher

- n 2 oder 3 ist,
- $R_4$ Alkyl mit 1-4 C-Atomen oder Alkoxyalkyl mit 2-5 C-Atomen ist,

$R_5$ H, Alkoxy mit 1-4 C-Atomen, Halogen, NO oder $NO_2$ ist und
L ein luminophorer Rest in para- oder meta-Stellung zum Stickstoff ist,

oder die Gruppe mit der allgemeinen Formel IV

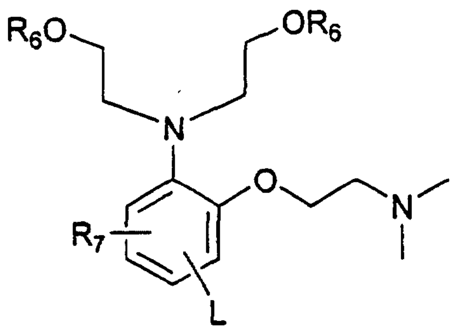

(IV)

ist, in welcher

$R_6$ Alkyl mit 1-3 C-Atomen oder Phenyl ist,
$R_7$ H, Alkoxy mit 1-4 C-Atomen, Halogen, NO oder $NO_2$ ist und
L ein luminophorer Rest in para- oder meta-Stellung zum Stickstoff ist.

**2.** Verbindung gemäß Anspruch 1 mit einer Gruppe der allgemeinen Formel (II), dadurch gekennzeichnet, daß $R_1$ Alkyl mit 4 C-Atomen, Alkoxyalkyl mit 3-4 C-Atomen oder Aryloxyalkyl, dessen Alkylrest 2 C-Atome aufweist, ist.

**3.** Verbindung nach Anspruch 1 oder 2 mit einer Gruppe der allgemeinen Formel (II), dadurch gekennzeichnet, daß $R_2$ Alkyl mit 1-4 C-Atomen ist.

**4.** Verbindung nach Anspruch 3 mit einer Gruppe der allgemeinen Formel (II), dadurch gekennzeichnet, daß $R_2$ Methyl ist.

**5.** Verbindung nach einem der Ansprüche 1 bis 4 mit einer Gruppe der allgemeinen Formel (II), dadurch gekennzeichnet, daß $R_3$ in para-Stellung zum o-Anisidin-Sauerstoff angeordnet ist.

**6.** Verbindung nach einem der Ansprüche 1 bis 5 mit einer Gruppe der allgemeinen Formel (II), dadurch gekennzeichnet, daß $R_3$ H oder Methoxy ist.

**7.** Verbindung nach Anspruch 1 mit einer Gruppe der allgemeinen Formel (III), dadurch gekennzeichnet, daß n = 2 ist.

**8.** Verbindung nach Anspruch 1 oder 7 mit einer Gruppe der allgemeinen Formel (III), dadurch gekennzeichnet, daß $R_4$ Alkyl mit 1-2 C-Atomen ist.

**9.** Verbindung nach einem der Ansprüche 1, 7 oder 8 mit einer Gruppe der allgemeinen Formel (III), dadurch gekennzeichnet, daß $R_5$ in para-Stellung zum o-Anisidin-Sauerstoff angeordnet ist.

**10.** Verbindung nach einem der Ansprüche 1 oder 7 bis 9 mit einer Gruppe der allgemeinen Formel (III), dadurch gekennzeichnet, daß $R_5$ H oder Ethoxy oder Cl ist.

**11.** Verbindung nach Anspruch 1 mit einer Gruppe der allgemeinen Formel (IV), dadurch gekennzeichnet, daß $R_6$ Methyl ist.

**12.** Verbindung nach Anspruch 1 oder 11 mit einer Gruppe der allgemeinen Formel (IV), dadurch gekennzeichnet, daß $R_7$ in para-Stellung zum o-Anisidin-Sauerstoff angeordnet ist.

**13.** Verbindung nach einem der Ansprüche 1, 11 oder 12 mit einer Gruppe der allgemeinen Formel (IV), dadurch

gekennzeichnet, daß $R_7$ H ist.

**14.** Verbindung nach einem der Ansprüche 1 bis 13, dadurch gekennzeichnet, daß L in para-Stellung zum Stickstoff angeordnet ist.

**15.** Verbindung nach einem der Ansprüche 1 bis 14, dadurch gekennzeichnet, daß diese in Form des Dikaliumsalzes vorliegt.

**16.** Optischer Sensor zur Bestimmung von Calciumionen in einer Probe, welcher Sensor eine Matrix, die eine Verbindung mit einem luminophoren Rest und einem ionophoren Rest aufweist, besitzt, dadurch gekennzeichnet, daß als Verbindung eine Verbindung gemäß einem der Ansprüche 1 bis 15 eingesetzt wird.

**17.** Verfahren zur Bestimmung von Calciumionen in einer Probe, wobei die Probe mit einer Verbindung, die einen luminophoren Rest und einen ionophoren Rest aufweist, in zumindest indirekten Kontakt gebracht wird, welcher ionophore Rest mit den in der Probe enthaltenen Calciumionen reagiert, wobei der luminophore Rest seine Lumineszenzeigenschaften ändert, wonach die Lumineszenz gemessen und mit dem Meßergebnis die Calciumionen bestimmt werden, dadurch gekennzeichnet, daß als Verbindung eine Verbindung gemäß einem der Ansprüche 1 bis 15 eingesetzt wird.

**18.** Verwendung einer Verbindung gemäß einem der Ansprüche 1 bis 15 in einem optischen Sensor zur Bestimmung von Calciumionen in einer Probe.

## Claims

**1.** A compound having the general formula I

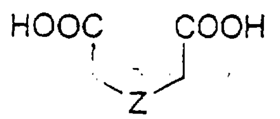

(I)

including its salts, wherein Z is either the group having the general formula II

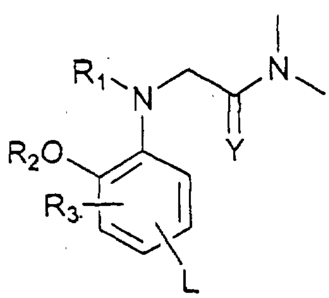

(II)

wherein

- $R_1$ is alkyl having 1-4 C atoms, alkoxy alkyl having 2-5 C atoms, or aryl-oxy alkyl whose alkyl residue has 1-4 C atoms,
- $R_2$ is alkyl having 1-4 C atoms or alkoxy alkyl having 2-5 C atoms,
- $R_3$ is H, alkoxy having 1-4 C atoms, halogen, NO, or $NO_2$,
- Y is $H_2$ or O, and
- L is a luminophore residue in para- or metaposition to the nitrogen,

or the group having the general formula III

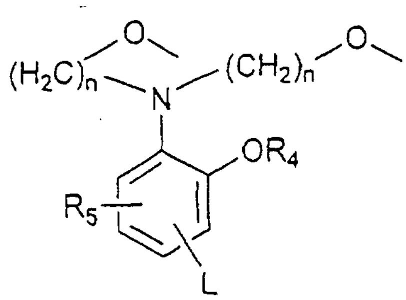

(III)

wherein

n is 2 or 3,
$R_4$ is alkyl having 1-4 C atoms or alkoxy alkyl having 2-5 C atoms,
$R_5$ is H, alkoxy having 1-4 C atoms, halogen, NO, or $NO_2$, and
L is a luminophore residue in para- or metaposition to the nitrogen,

or the group having the general formula IV

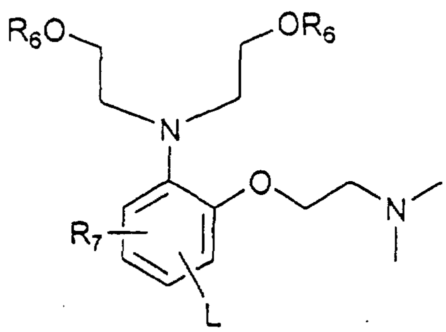

(IV)

wherein

$R_6$ is alkyl having 1-3 C atoms or phenyl,
$R_7$ is H, alkoxy having 1-4 C atoms, halogen, NO, or $NO_2$, and
L is a luminophore residue in para- or metaposition to the nitrogen.

2. A compound according to claim 1, having a group of the general formula (II), characterized in that $R_1$ is alkyl having 4 C atoms, alkoxy alkyl having 3-4 C atoms, or aryl-oxy alkyl whose alkyl residue has 2 C atoms.

3. A compound according to claim I or 2, having a group of the general formula (II), characterized in that $R_2$ is alkyl having 1-4 C atoms.

4. A compound according to claim 3, having a group of the general formula (II), characterized in that $R_2$ is methyl.

5. A compound according to anyone of claims 1 to 4, having a group of the general formula (II), characterized in that $R_3$ is located in paraposition to the o-anisidine oxygen.

6. A compound according to anyone of claims 1 to 5, having a group of the general formula (II), characterized in that $R_3$ is H or methoxy.

7. A compound according to claim 1, having a group of the general formula (III), characterized in that n = 2.

8. A compound according to claim 1 or 7, having a group of the general formula (III), characterized in that $R_4$ is alkyl

having 1-2 C atoms,

**9.** A compound according to anyone of claims 1, 7, or 8, having a group of the general formula (III), characterized in that $R_5$ is located in paraposition to the o-anisidine oxygen.

**10.** A compound according to anyone of claims 1 or 7 to 9, having a group of the general formula (III), characterized in that $R_5$ is H or ethoxy or Cl.

**11.** A compound according to claim 1, having a group of the general formula (IV), characterized in that $R_6$ is methyl.

**12.** A compound according to claim 1 or 11, having a group of the general formula (IV), characterized in that $R_7$ is located in paraposition to the o-anisidine oxygen.

**13.** A compound according to anyone of claims 1, 11, or 12, having a group of the general formula (IV), characterized in that $R_7$ is H.

**14.** A compound according to anyone of claims 1 to 13, characterized in that L is located in paraposition to the nitrogen.

**15.** A compound according to anyone of claims 1 to 14, characterized in that said compound is in the form of the dipotassium salt.

**16.** An optical sensor for determining calcium ions in a sample, which sensor has a matrix which has a compound having a luminophore residue and an ionophore residue, characterized in that a compound according to anyone of claims 1 to 15 is used as said compound.

**17.** A process for determining calcium ions in a sample, wherein the sample is brought into at least indirect contact with a compound having a luminophore residue and an ionophore residue, which ionophore residue reacts with the calcium ions contained in the sample, the luminophore residue changing its luminescence properties, whereupon luminescence is measured and, by the measurement result, the calcium ions are determined, characterized in that a compound according to anyone of claims 1 to 15 is used as said compound.

**18.** A use of a compound according to anyone of claims 1 to 15 in an optical sensor for determining calcium ions in a sample.

**Revendications**

**1.** Composé de formule générale I

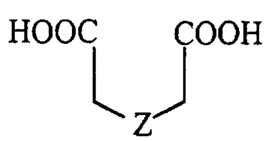

(I)

y compris ses sels, où Z est le groupe de formule générale II

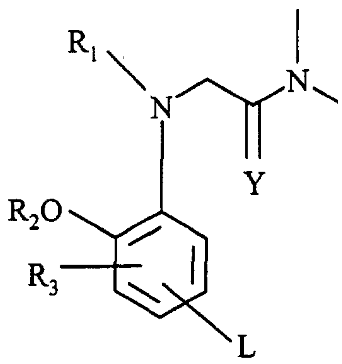

(II)

où

$R_1$ est alkyle avec 1 à 4 atomes de carbone, alcoxyalkyle avec 2 à 5 atomes de carbone ou aryloxyalkyle dont le reste alkyle comporte 1 à 4 atomes de carbone,
$R_2$ est alkyle avec 1 à 4 atomes de carbone ou alcoxyalkyle avec 2 à 5 atomes de carbone,
$R_3$ est H, alcoxy avec 1 à 4 atomes de carbone, halogène, NO ou $NO_2$,
Y est $H_2$ ou O et
L est un reste luminophore en position para ou méta par rapport à l'azote,

ou le groupe de formule générale III

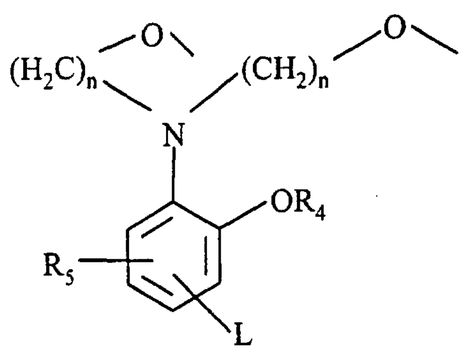

(III)

où

n est 2 ou 3,
$R_4$ est alkyle avec 1 à 4 atomes de carbone ou alcoxyalkyle avec 2 à 5 atomes de carbone,
$R_5$ est H, alcoxy avec 1 à 4 atomes de carbone, halogène, NO ou $NO_2$ et
L est un reste luminophore en position para ou méta par rapport à l'azote

ou le groupe de formule générale IV

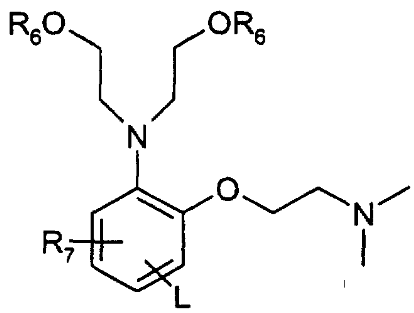

(IV)

où

$R_6$ est alkyle avec 1 à 3 atomes de carbone ou phényle,
$R_7$ est H, alcoxy avec 1 à 4 atomes de carbone, halogène, NO ou $NO_2$ et
L est un reste luminophore en position para ou méta par rapport à l'azote

**2.** Composé selon la revendication 1 avec un groupe de formule générale (II), caractérisé en ce que $R_1$ est alkyle avec 4 atomes de carbone, alcoxyalkyle avec 3 à 4 atomes de carbone ou aryloxyalkyle dont le reste alkyle comporte 2 atomes de carbone.

**3.** Composé selon la revendication 1 ou 2, avec un groupe de formule générale (II), caractérisé en ce que $R_2$ est alkyle avec 1 à 4 atomes de carbone.

**4.** Composé selon la revendication 3 avec un groupe de formule générale (II), caractérisé en ce que $R_2$ est méthyle.

**5.** Composé selo l'une des revendications 1 à 4 avec un groupe de formule générale (II), caractérisé en ce que $R_3$ est disposé en position para par rapport à l'oxygène de la o-anisidine.

**6.** Composé selon l'une des revendications 1 à 5 avec un groupe de formule générale (II) caractérisé en ce que $R_3$ est H ou méthoxy.

**7.** Composé selon la revendication 1 avec un groupe de formule générale (III), caractérisé en ce que n = 2.

**8.** Composé selon la revendication 1 ou 7 avec un groupe de formule générale (III), caractérisé en ce que $R_4$ est alkyle avec 1 à 2 atomes de carbone.

**9.** Composé selon l'une des revendications 1, 7 ou 8 avec un groupe de formule générale (III), caractérisé en ce que $R_5$ est disposé en position para par rapport à l'oxygène de la o-anisidine.

**10.** Composé selon l'une des revendications 1 ou 7 à 9 avec un groupe de formule générale (III), caractérisé en ce que $R_5$ est H ou éthoxy ou Cl.

**11.** Composé se on la revendication 1 avec un groupe de formule générale (IV), caractérisé en ce que $R_6$ est méthyle.

**12.** Composé selon la revendication 1 ou 11 avec un groupe de formule générale (IV), caractérisé en ce que $R_7$ est disposé en position para par rapport à l'oxygène de la o-anisidine.

**13.** Composé selon l'une des revendications 1, 11 ou 12 avec un groupe de formule générale (IV), caractérisé en ce que $R_7$ est H.

**14.** Composé selon l'une des revendications 1 à 13, caractérisé en ce que L est disposé en position para par rapport à l'azote.

**15.** Composé selon l'une des revendications 1 à 14, caractérisé en ce qu'il est sous forme du sel de dipotassium.

16. Détecteur optique pour déterminer les ions calcium dans un échantillon, lequel détecteur possède une matrice qui comporte un composé avec un reste luminophore et un reste ionophore, caractérisé en ce qu'un composé selon l'une des revendications 1 à 15 est utilisé comme composé.

17. Procédé pour déterminer les ions calcium dans un échantillon, où l'échantillon est mis en contact au moins indirect avec un composé qui comporte un reste luminophore et un reste ionophore, lequel reste ionophore réagit avec les ions calcium contenus dans l'échantillon, le reste luminophore modifiant ses propriétés de luminescence, après quoi la luminescence est mesurée et les ions calcium sont déterminés avec le résultat de la mesure, caractérisé en ce qu'un composé selon l'une des revendications 1 à 15 est utilisé comme composé.

18. Utilisation d'un composé selon l'une des revendications 1 à 15 dans un détecteur optique pour déterminer les ions calcium dans un échantillon.

# Fig. 1

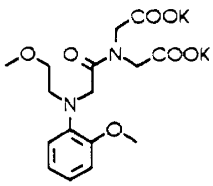

X1

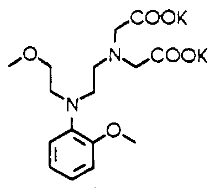

X2

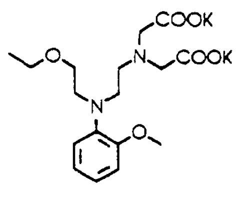

X3

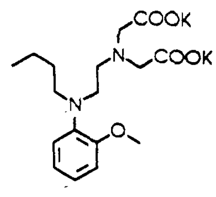

X4

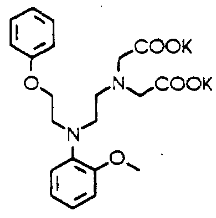

X5

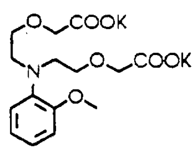

X6

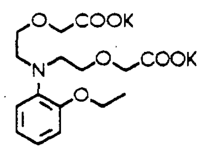

X7

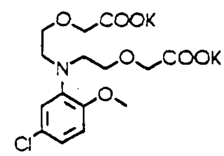

X8

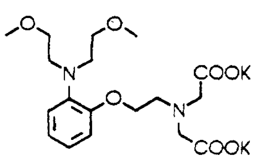

X9

Fig. 2A

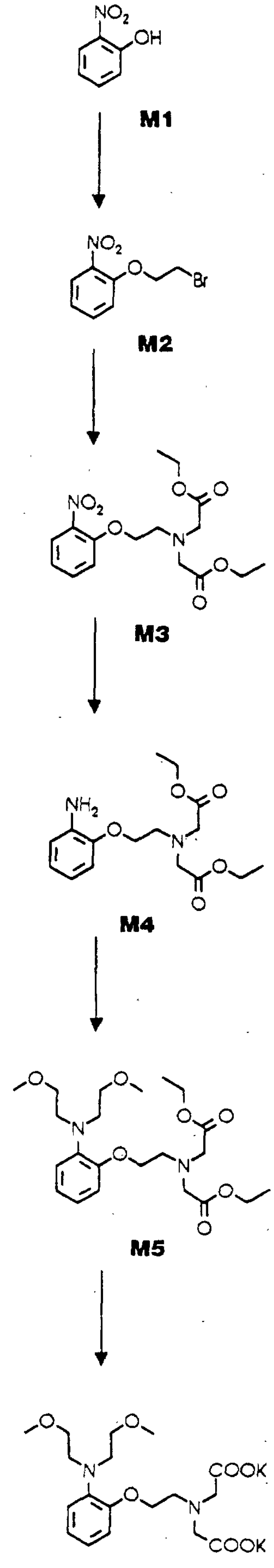

M1

M2

M3

M4

M5

X9

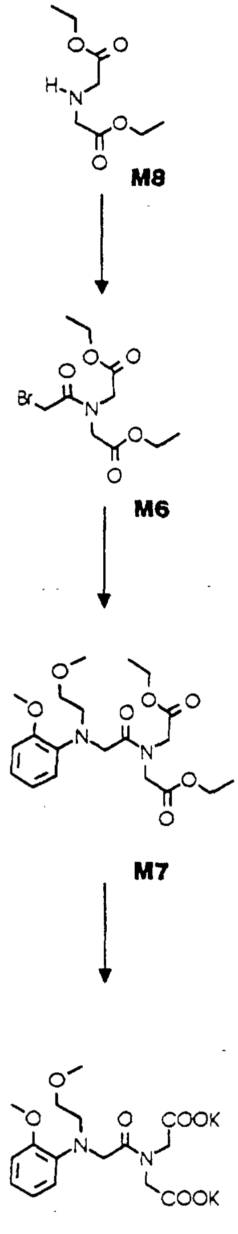

M8

M6

M7

X1

**Fig. 2B**

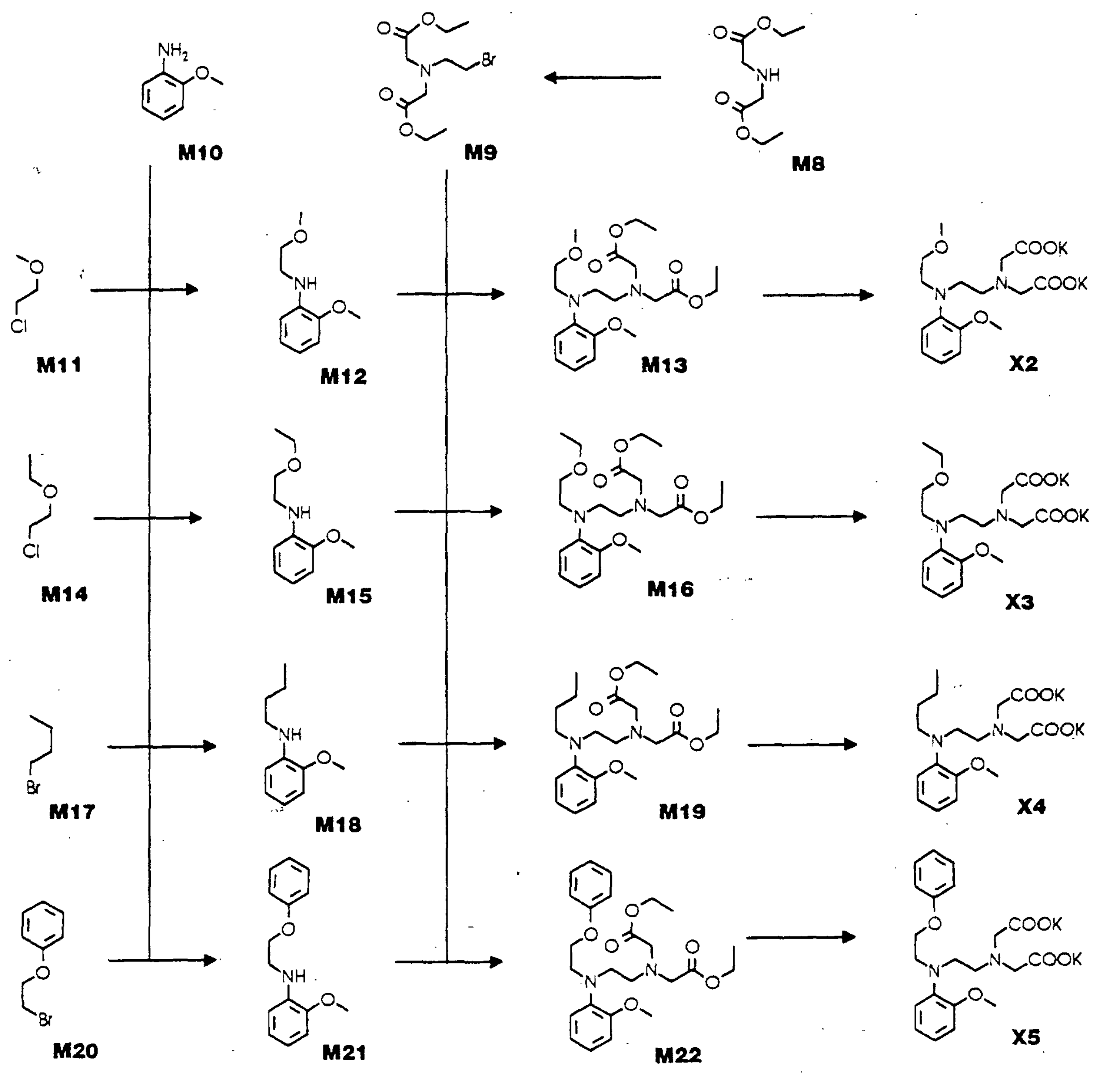

M10 M9 M8

M11 M12 M13 X2

M14 M15 M16 X3

M17 M18 M19 X4

M20 M21 M22 X5

# Fig. 2C

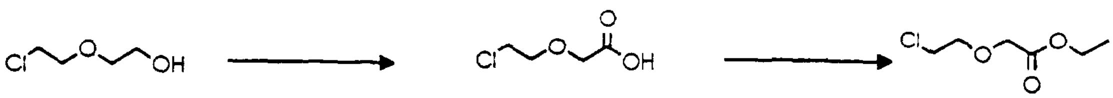

M23 M24 M25

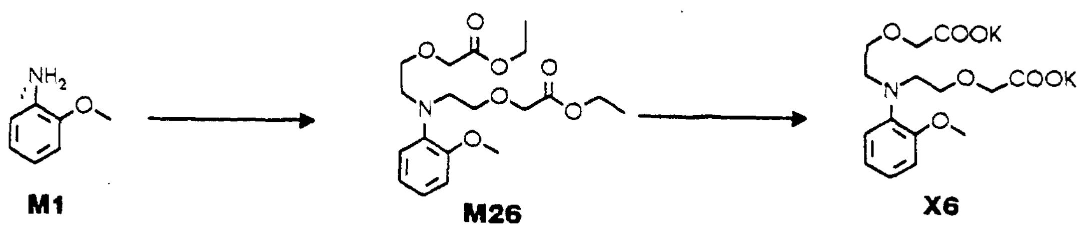

M1 M26 X6

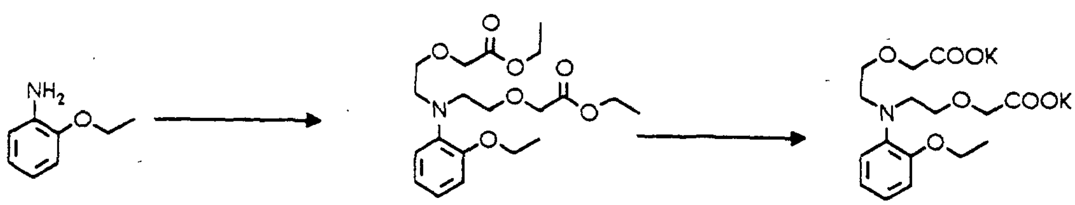

M27 M28 X7

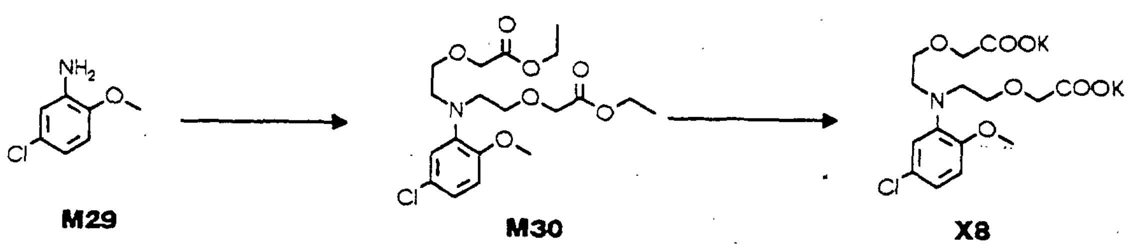

M29 M30 X8

# Fig. 3A

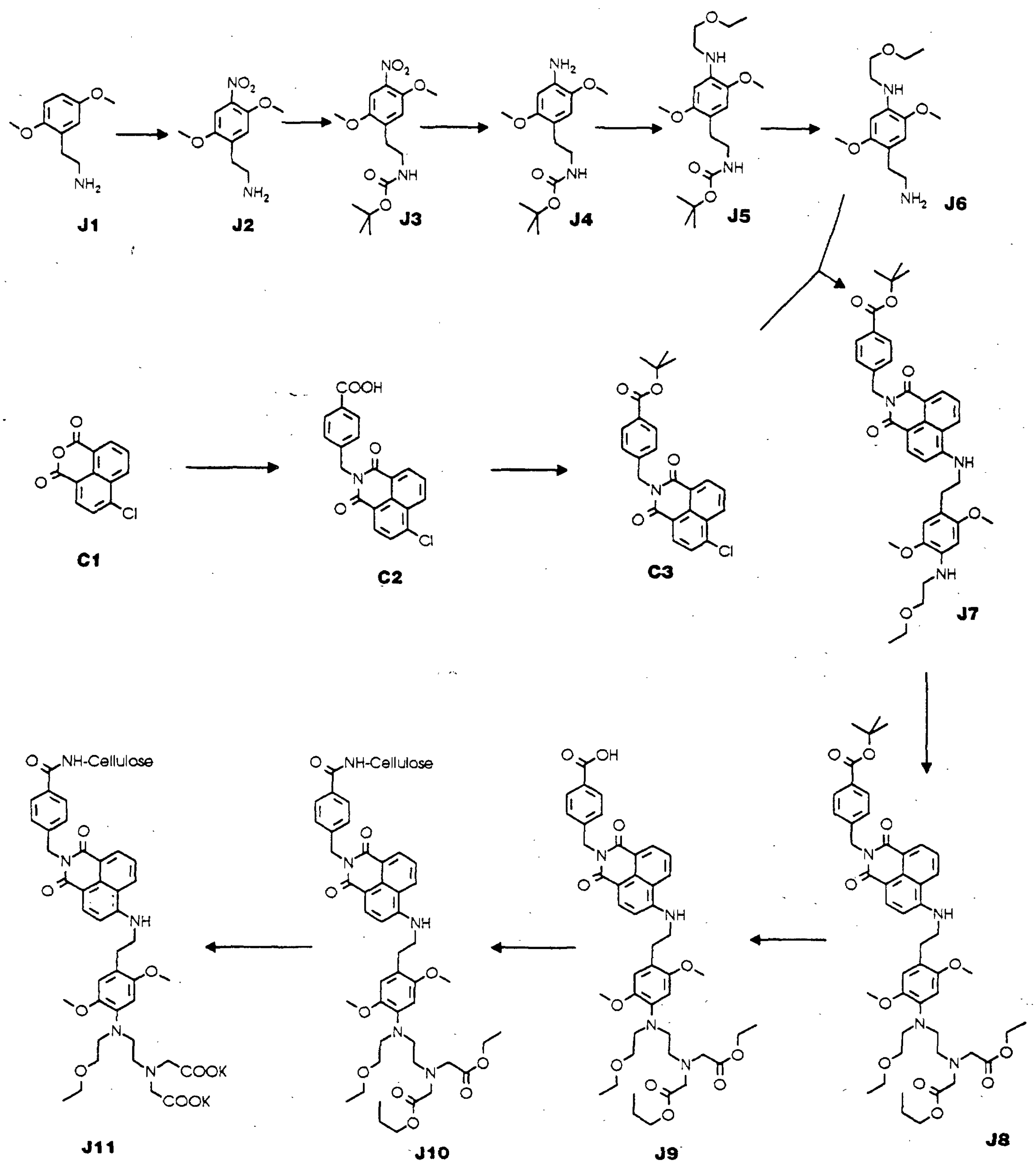

J1 J2 J3 J4 J5 J6

C1 C2 C3 J7

J11 J10 J9 J8

# Fig. 3B

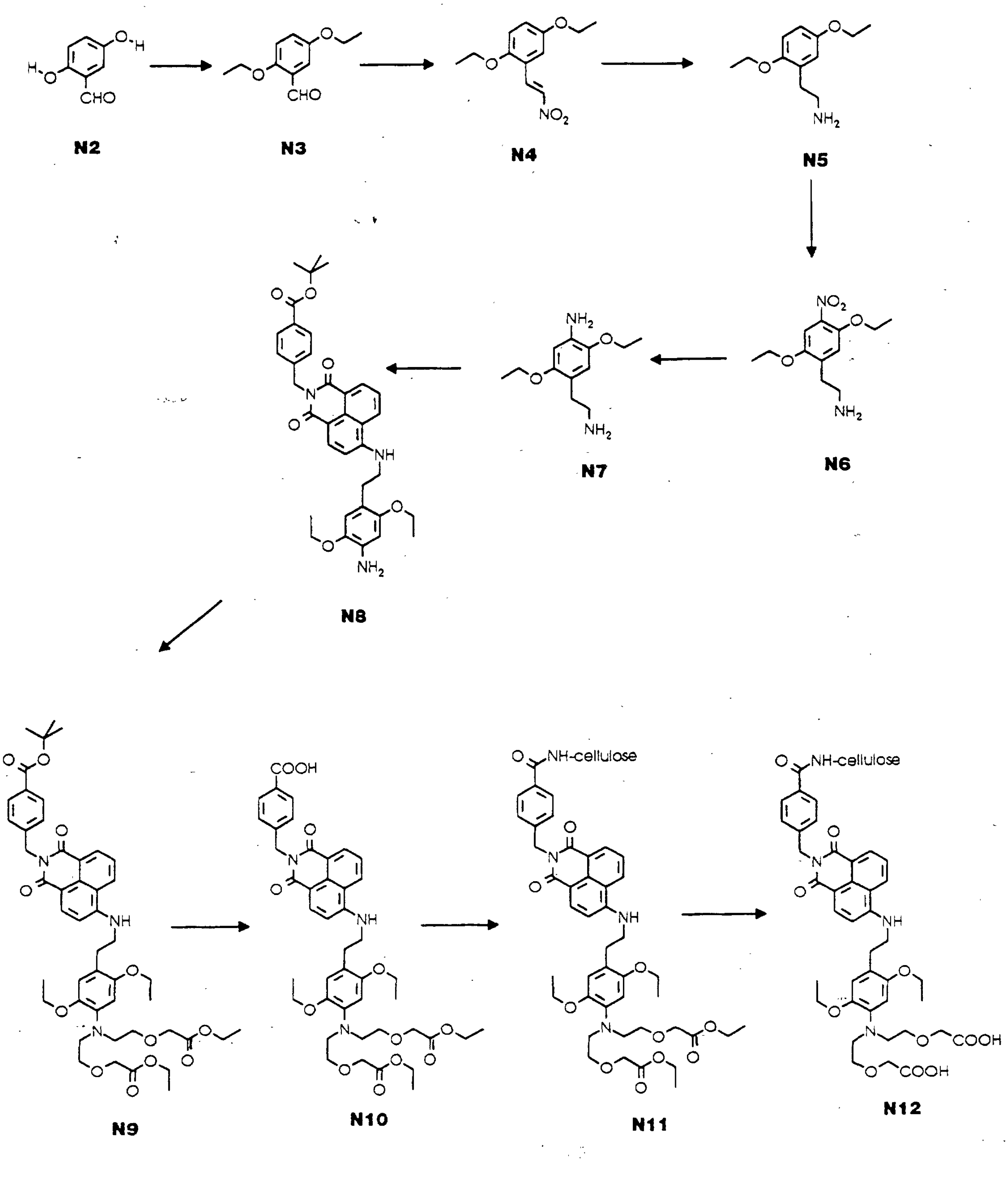

N2 N3 N4 N5

N6 N7 N8

N9 N10 N11 N12

Fig. 4

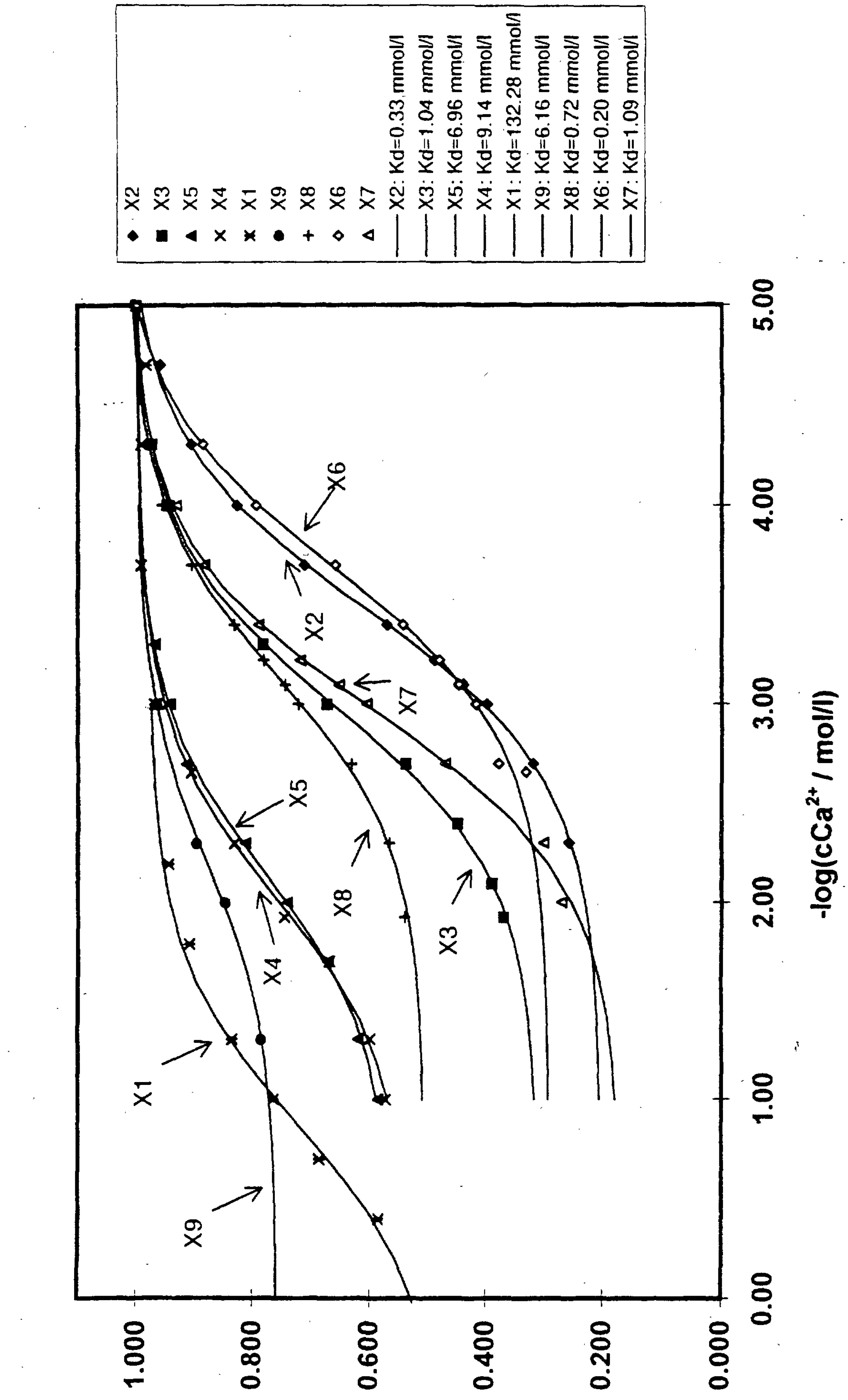
X2
X3
X5
X4
X1
X9
X8
X6
X7
X2: Kd=0.33 mmol/l
X3: Kd=1.04 mmol/l
X5: Kd=6.96 mmol/l
X4: Kd=9.14 mmol/l
X1: Kd=132.28 mmol/l
X9: Kd=6.16 mmol/l
X8: Kd=0.72 mmol/l
X6: Kd=0.20 mmol/l
X7: Kd=1.09 mmol/l
1.000
0.800
0.600
0.400
0.200
0.000
0.00
1.00
2.00
3.00
4.00
5.00
-log(cCa2+ / mol/l)

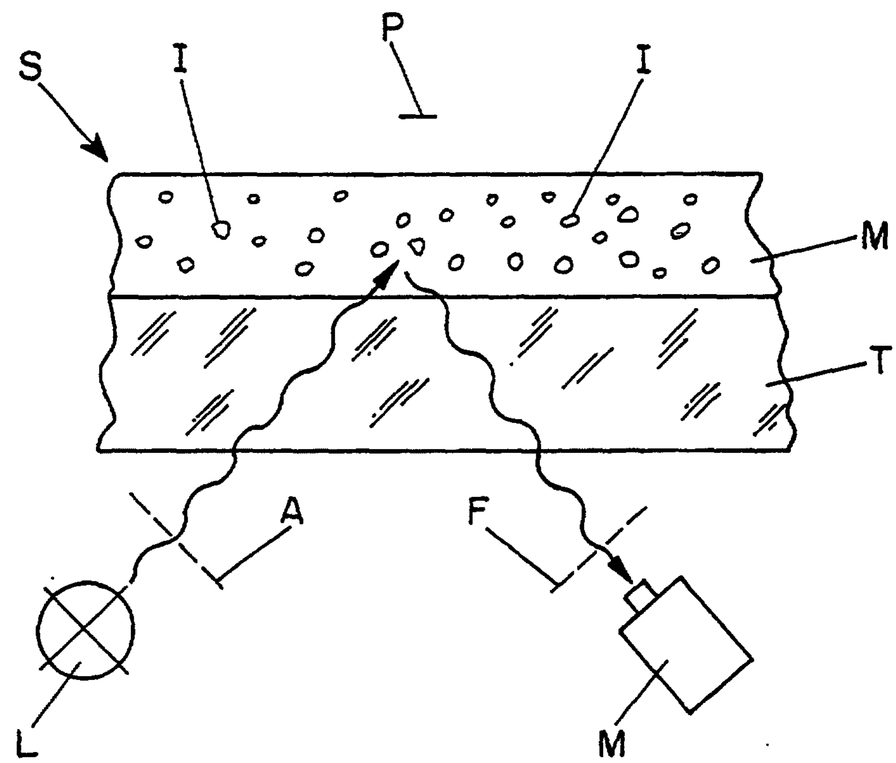


FIG. 5

Fig. 6

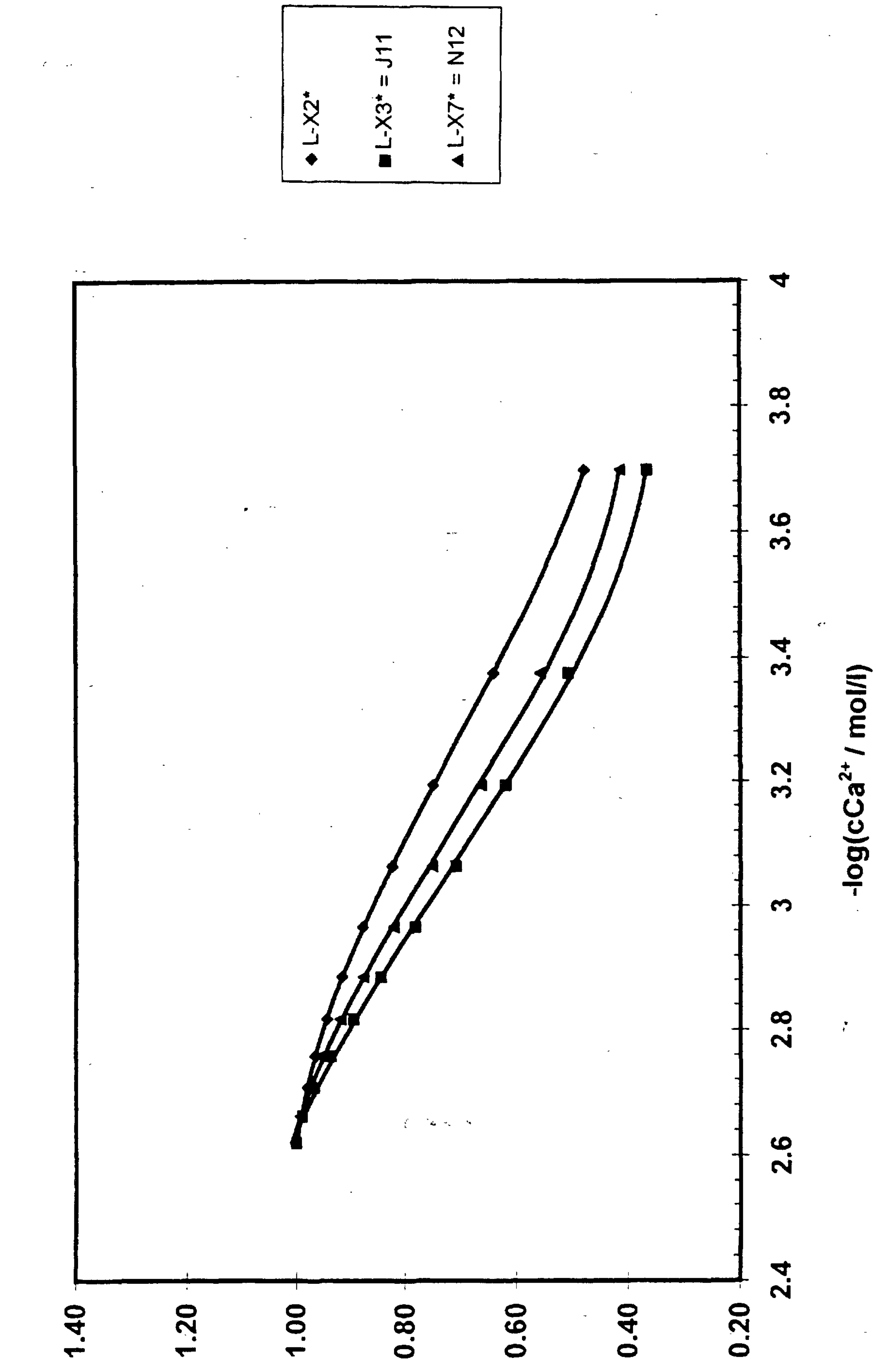

L-X2*
L-X3* = J11
L-X7* = N12
1.40
1.20
1.00
0.80
0.60
0.40
0.20
2.4
2.6
2.8
3
3.2
3.4
3.6
3.8
4
-log(cCa2+ / mol/l)

Fig. 7

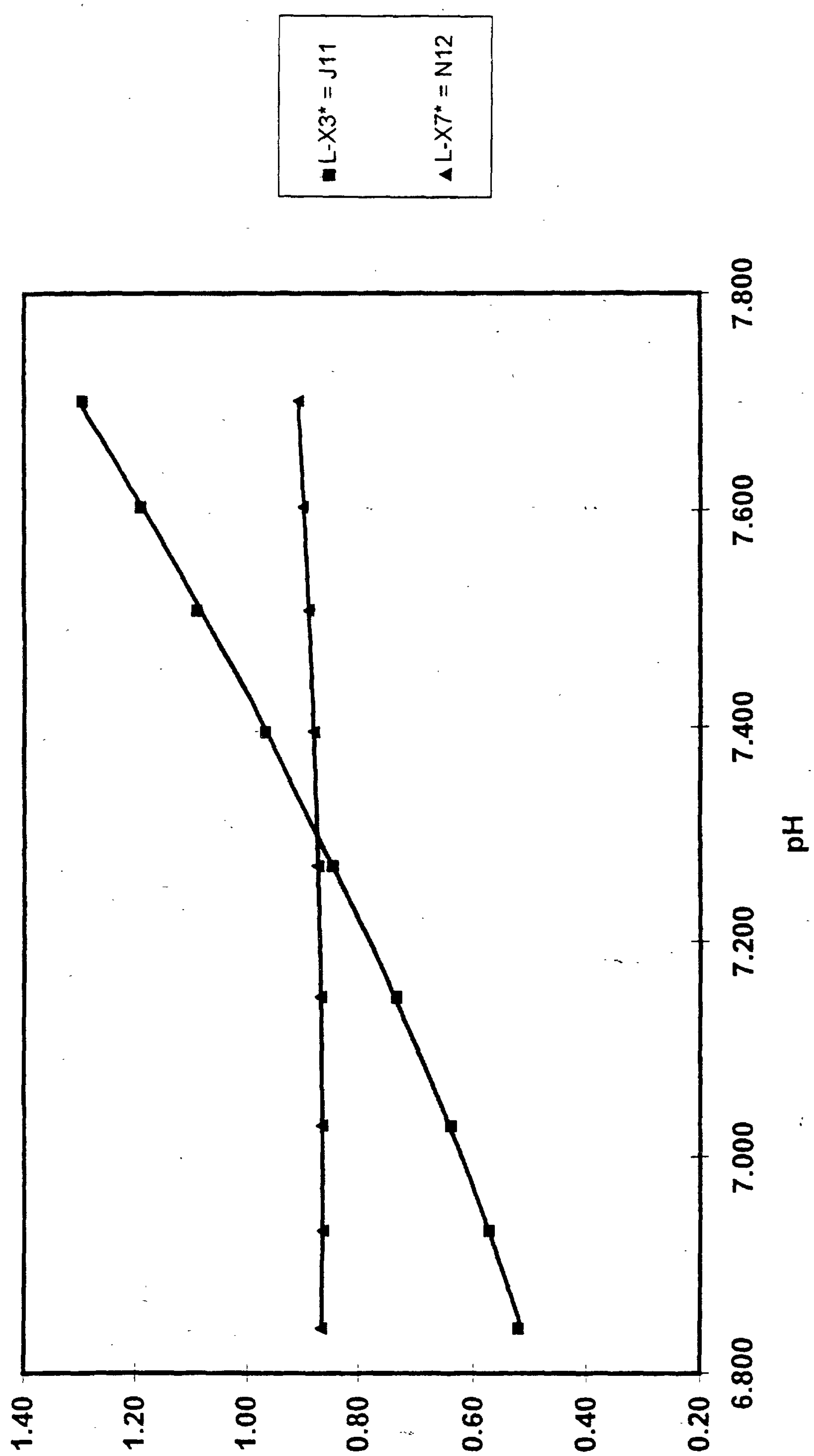
L-X3* = J11
L-X7* = N12
1.40
1.20
1.00
0.80
0.60
0.40
0.20
6.800
7.000
7.200
7.400
7.600
7.800
pH